# EUROPEAN PATENT APPLICATION

(11) **EP 4 324 854 A1**
(43) Date of publication of application: **21.02.2024**
(21) Application number: 22787605.9
(22) Date of filing: 14.04.2022
(51) Int. Cl.: C07K 16/46, C12N 15/13, A61K 39/395, A61K 45/00, A61P 35/00, A61P 35/02, G01N 33/68

(54) **USE OF ANTIBODY IN ANTI-TUMOR TREATMENT**

(30) Priority: 14.04.2021 CN 202110404963
(71) Applicant: Akeso Pharmaceuticals, Inc., Guangzhou, Guangdong 510799 (CN); AD Pharmaceutical Co., Ltd., Guangzhou Guangdong 510530 (CN)
(72) Inventor: XIA, Yu, Zhongshan Guangdong 528437 (CN); WANG, Zhongmin Maxwell, Zhongshan Guangdong 528437 (CN); LI, Baiyong, Zhongshan Guangdong 528437 (CN)
(74) Representative: Nederlandsch Octrooibureau
(86) International application number: PCT/CN2022/086870
(87) International publication number: WO 2022/218383

(57) **Abstract**

The present invention relates to a bispecific antibody against CTLA4 and PD-1, wherein the antibody is used for treating tumors, is administered in combination with an anti-VEGFR2 monoclonal antibody or an anti-VEGF monoclonal antibody,
and comprises
a first protein functional region targeting PD-1 and
a second protein functional region targeting CTLA4,
the first protein functional region being an immunoglobulin and the second protein functional region being a single-chain antibody, or the first protein functional region being a single-chain antibody and the second protein functional region being an immunoglobulin.

## Description

### TECHNICAL FIELD

The present invention relates to the field of molecular immunology, and particularly relates to use of an anti-vascular endothelial growth factor receptor VEGFR2 monoclonal antibody or an anti-VEGF monoclonal antibody in combination with an anti-PD-1/CTLA-4 bispecific antibody in treating a tumor.

### BACKGROUND

Tumor is a serious health-threatening disease, and there is an urgent need to develop effective treatments and drugs.

Vascular endothelial growth factor receptor 2 (VEGFR2) is a receptor that specifically acts on a growth factor of a vascular endothelial cell, and is also called KDR or FLK1. There are mainly 5 VEGFR types currently discovered: VEGFR-1, VEGFR-2, VEGFR-3, NP-1, and NP-2, wherein VEGFR-1 and VEGFR2 are mainly present in vascular endothelial cells, and VEGFR3 is mainly present in lymphatic endothelial cells. NP-1 and NP-2 are expressed not only in endothelial cells but also in some tumor cells. However, VEGF exerts its biological activities such as promoting the proliferation of vascular endothelial cells mainly by binding to VEGFR2.

The formation of new blood vessels plays an important role in a variety of diseases in humans, such as retinopathy, arthritis, endometriosis, and the like. The growth of a tumor is usually accompanied by the formation of new blood vessels. As early as 1971, J.Folkman proposed that the growth and metastasis of a tumor can be inhibited by blocking the angiogenesis of the tumor. In recent years, more and more malignant tumors are found to be related to vascular endothelial growth factors and receptor families thereof in development, metastasis, and prognosis. Therefore, again, anti-tumor therapies targeting VEGF and receptors thereof are of interest, and among the receptor families, VEGFR-2 as a target is the most widely studied.

PD-1 is a key immune checkpoint receptor expressed by activated T lymphocytes and B lymphocytes and mediates immunosuppression, and its ligands include at least PD-L1 and PD-L2. Anti-PD-1 antibodies specifically bind to programmed death receptor-1 (PD-1) and block the inhibitory PD-1/PD-1 ligand pathway (Topalian et al., (2012a) N Engl J Med 366: 2443-54).

PD-L1 (programmed death-ligand 1), also known as CD274 or B7-H1, is a 40 kDa type 1 transmembrane protein encoded by the CD274 gene, and is a ligand of PD-1. PD-L1 is widely distributed not only on leucocytes and nonhematopoietic cells in lymphoid and non-lymphoid tissues, but also in various cancer cells, and it is highly expressed on the surface of various tumor cells, and the malignant degree and poor prognosis of tumors are closely related to the expression level of PD-L1. Both PD-L1 and PD-1 belong to the immunoglobulin superfamily and have two extracellular Ig domains. The binding interface of PD-L1 to programmed death receptor-1 (PD-1) and B7-1 (CD80) is on an IgV-like domain (Lin et al., (2008) PNAS 105: 3011-3016). PD-L1 contains a conserved short intracellular tail (about 30 amino acids), and PD-1 contains two cytoplasmic tyrosine-based signaling motifs, namely an immunoreceptor tyrosine-based inhibitory motif (ITIM) and an immunoreceptor tyrosine-based switch motif (ITSM). Following T cell stimulation, PD-1 recruits the tyrosine phosphatase SHP-2 to the ITSM motif in its cytoplasmic tail, resulting in dephosphorylation of effector molecules (such as CD3ζ, PKCθ and ZAP70) involved in the CD3+ T cell signaling cascade (Freeman et al., (2000) J Exp Med 192: 1027-34; Latchman et al., (2001) Nat Immunol 2: 261-8; Carter et al., (2002) Eur J Immunol 32: 634-43). Clinical data have suggested that high tumor expression of PD-L1 is associated with increased tumor invasiveness and poor prognosis.

PD-1/PD-L1 is an important specific immune checkpoint. The formation of the PD-1/PD-L1 complex transmits inhibitory signals and negatively regulates T cell immune response; it inhibits TCR-mediated T cell activation, cytokine production, and T cell proliferation (Fife et al., (2011) Nature Immunology 10: 1185-1193), induces exhaustion or anergy among cognate antigen-specific T cells (Hofmeyer et al., (2011) Journal of Biomedicine and Biotechnology 2011: 1-9), promotes the differentiation of Th1 cells into Foxp3+ regulatory T cells (Armanath et al., (2011) Science TransMed 3: 1-13; Francisco et al., (2009) J.Exp.Med.206: 3015-3029), and induces apoptosis of effector T cells. The disruption of PD-L1 genes results in an upregulated T cell response and the production of autoreactive T cells (Latchman et al., (2004) PNAS, 101:10691-10696). The blockade of PD-1 or PD-L1 by antibody results in elevated anti-tumor immunity (Iwai et al., (2002) PNAS, 99:12293-12297). CTLA-4 is also one of the representative specific immune checkpoint proteins. CTLA-4 is the first target to be clinically applied. The anti-CTLA-4 antibody can promote T cell activation and improve the anti-tumor effect thereof. Ipilimumab is the first CTLA-4 immune checkpoint inhibitor demonstrated to improve overall survival of patients with metastatic melanoma previously treated and approved by FDA. Current studies indicate that CTLA-4 inhibits the response of T cells mainly via two pathways. One is by competitive binding to B7 with CD28, or recruitment of phosphatases to the intracellular domain of the CTLA-4, thereby reducing the signal of TCR (T cell receptor) and CD28. Another is to reduce the expression level of CD80 and CD86 on antigen-presenting cells (APCs) or to remove them from the APCs by transendocytosis, thus reducing CD28-involving T cell activation. In addition, CTLA-4 also mediates the binding of dendritic cells to CD80/CD86 and induces the expression of tryptophan-degrading enzyme IDO, resulting in inhibition of TCR. CTLA-4 antibodies reduce Treg and activate TCR by binding to CTLA-4.

The combined use of PD-1 antibodies and CTLA-4 (cytotoxic T lymphocyte-associated protein 4) antibody medicament shows better efficacy on a plurality of tumors than the PD-1 antibody alone. However, the improvement of the efficacy of the combined use is always accompanied by higher toxicity, and the application is greatly limited.

Bevacizumab, an anti-angiogenesis targeted drug, hinders the growth of blood vessels and inhibits the metastasis of tumors by binding to a vascular endothelial growth factor (VEGF), which is a broad-spectrum anticancer drug. Since the launch, bevacizumab has been approved primarily for the treatment of various types of cancer, such as brain cancer (e.g., aggressive brain cancer, e.g., glioblastoma), kidney cancer, lung cancer (e.g., metastatic non-squamous non-small cell lung cancer), colon cancer, rectal cancer, cervical cancer (e.g., metastatic cervical cancer), endometrial cancer, ovarian cancer (e.g., advanced ovarian cancer) or fallopian tube cancer, kidney cancer, and the like.

### SUMMARY

The inventors have found that an anti-CTLA4-anti-PD-1 bispecific antibody in combination with an anti-VEGFR2 monoclonal antibody or in combination with an anti-VEGF monoclonal antibody can effectively prevent and treat tumors, and has low toxic and side effects. The present invention is detailed below.

Specifically, the present invention relates to:
1. An anti-CTLA4-anti-PD-1 bispecific antibody, wherein the anti-CTLA4-anti-PD-1 bispecific antibody is preferably used for treating a tumor; preferably, the anti-CTLA4-anti-PD-1 bispecific antibody is administered in combination with component A, wherein the component A is selected from an anti-VEGFR2 monoclonal antibody or an antigen-binding fragment thereof, an anti-VEGF monoclonal antibody or an antigen-binding fragment thereof, or a combination thereof, wherein
   (1) the anti-CTLA4-anti-PD-1 bispecific antibody comprises:
      a first protein functional region targeting PD-1 and
      a second protein functional region targeting CTLA4,
      wherein the first protein functional region is an immunoglobulin, and the second protein functional region is a single chain antibody; or, the first protein functional region is a single chain antibody, and the second protein functional region is an immunoglobulin;
      wherein,
      the immunoglobulin comprises HCDR1-HCDR3 (preferably HCDR1-HCDR3 set forth in SEQ ID NOs: 27-29, respectively) in a heavy chain variable region set forth in SEQ ID NO: 14 and LCDR1-LCDR3 (preferably LCDR1-LCDR3 set forth in SEQ ID NOs: 30-32, respectively) in a light chain variable region set forth in SEQ ID NO: 16; the single chain antibody comprises HCDR1-HCDR3 (preferably HCDR1-HCDR3 set forth in SEQ ID NOs: 33-35, respectively) in a heavy chain variable region set forth in SEQ ID NO: 2 and LCDR1-LCDR3 (preferably LCDR1-LCDR3 set forth in SEQ ID NOs: 36-38, respectively) in a light chain variable region set forth in SEQ ID NO: 4; or,
      the immunoglobulin comprises HCDR1-HCDR3 (preferably HCDR1-HCDR3 set forth in SEQ ID NOs: 33-35, respectively) in a heavy chain variable region set forth in SEQ ID NO: 2 and LCDR1-LCDR3 (preferably LCDR1-LCDR3 set forth in SEQ ID NOs: 36-38, respectively) in a light chain variable region set forth in SEQ ID NO: 4; the single chain antibody comprises HCDR1-HCDR3 (preferably HCDR1-HCDR3 set forth in SEQ ID NOs: 27-29, respectively) in a heavy chain variable region set forth in SEQ ID NO: 14 and LCDR1-LCDR3 (preferably LCDR1-LCDR3 set forth in SEQ ID NOs: 30-32, respectively) in a light chain variable region set forth in SEQ ID NO: 16;
   (2) the anti-VEGFR2 monoclonal antibody comprises HCDR1-HCDR3 (preferably HCDR1-HCDR3 set forth in SEQ ID NOs: 53-55, respectively) in a heavy chain variable region set forth in SEQ ID NO: 50 and LCDR1-LCDR3 (preferably LCDR1-LCDR3 set forth in SEQ ID NOs: 56-58, respectively) in a light chain variable region set forth in SEQ ID NO: 52; or
   (3) the anti-VEGF monoclonal antibody comprises HCDR1-HCDR3 (preferably HCDR1-HCDR3 set forth in SEQ ID NOs: 63-65, respectively) in a heavy chain variable region set forth in SEQ ID NO: 60 and LCDR1-LCDR3 (preferably LCDR1-LCDR3 set forth in SEQ ID NOs: 66-68, respectively) in a light chain variable region set forth in SEQ ID NO: 62.
2. The anti-CTLA4-anti-PD-1 bispecific antibody according to item 1, wherein,
   (1) an amino acid sequence of the heavy chain variable region of the immunoglobulin is selected from SEQ ID NO: 14, SEQ ID NO: 18, or a variant thereof; an amino acid sequence of the light chain variable region of the immunoglobulin is selected from SEQ ID NO: 16, SEQ ID NO: 20, or a variant thereof; an amino acid sequence of the heavy chain variable region of the single chain antibody is selected from SEQ ID NO: 2, SEQ ID NO: 6, SEQ ID NO: 10, SEQ ID NO: 41, SEQ ID NO: 43, or a variant thereof; an amino acid sequence of the light chain variable region of the single chain antibody is selected from SEQ ID NO: 4, SEQ ID NO: 8, SEQ ID NO: 12, SEQ ID NO: 42, SEQ ID NO: 44, or a variant thereof;
      or,
      an amino acid sequence of the heavy chain variable region of the immunoglobulin is selected from SEQ ID NO: 2, SEQ ID NO: 6, SEQ ID NO: 10, SEQ ID NO: 41, SEQ ID NO: 43, or a variant thereof; an amino acid sequence of the light chain variable region of the immunoglobulin is selected from SEQ ID NO: 4, SEQ ID NO: 8, SEQ ID NO: 12, SEQ ID NO: 42, SEQ ID NO: 44, or a variant thereof; an amino acid sequence of the heavy chain variable region of the single chain antibody is selected from SEQ ID NO: 14, SEQ ID NO: 18, or a variant thereof; an amino acid sequence of the light chain variable region of the single chain antibody is selected from SEQ ID NO: 16, SEQ ID NO: 20, or a variant thereof;
   (2) an amino acid sequence of the heavy chain variable region of the anti-VEGFR2 monoclonal antibody is a sequence set forth in SEQ ID NO: 50 or a variant thereof; an amino acid sequence of the light chain variable region of the anti-VEGFR2 monoclonal antibody is a sequence set forth in SEQ ID NO: 52 or a variant thereof; or
   (3) an amino acid sequence of the heavy chain variable region of the anti-VEGF monoclonal antibody is a sequence set forth in SEQ ID NO: 60 or a variant thereof; an amino acid sequence of the light chain variable region of the anti-VEGF monoclonal antibody is a sequence set forth in SEQ ID NO: 62 or a variant thereof.
3. The anti-CTLA4-anti-PD-1 bispecific antibody according to any one of items 1 to 2, wherein the bispecific antibody is selected from any one of the following (1) - (20):
   (1) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 2 or a variant thereof, and an amino acid sequence of the light chain variable region of the single chain antibody is a sequence set forth in SEQ ID NO: 4 or a variant thereof;
   (2) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 6 or a variant thereof, and an amino acid sequence of the light chain variable region of the single chain antibody is a sequence set forth in SEQ ID NO: 8 or a variant thereof;
   (3) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 10 or a variant thereof, and an amino acid sequence of the light chain variable region of the single chain antibody is a sequence set forth in SEQ ID NO: 12 or a variant thereof;
   (4) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 2 or a variant thereof, and an amino acid sequence of the light chain variable region of the single chain antibody is a sequence set forth in SEQ ID NO: 4 or a variant thereof;
   (5) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 6 or a variant thereof, and an amino acid sequence of the light chain variable region of the single chain antibody is a sequence set forth in SEQ ID NO: 8 or a variant thereof;
   (6) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 10 or a variant thereof, and an amino acid sequence of the light chain variable region of the single chain antibody is a sequence set forth in SEQ ID NO: 12 or a variant thereof;
   (7) an amino acid sequence of the heavy chain variable region of the immunoglobulin is a sequence set forth in SEQ ID NO: 2 or a variant thereof, and an amino acid sequence of the light chain variable region of the immunoglobulin is a sequence set forth in SEQ ID NO: 4 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
   (8) an amino acid sequence of the heavy chain variable region of the immunoglobulin is a sequence set forth in SEQ ID NO: 2 or a variant thereof, and an amino acid sequence of the light chain variable region of the immunoglobulin is a sequence set forth in SEQ ID NO: 4 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
   (9) an amino acid sequence of the heavy chain variable region of the immunoglobulin is a sequence set forth in SEQ ID NO: 6 or a variant thereof, and an amino acid sequence of the light chain variable region of the immunoglobulin is a sequence set forth in SEQ ID NO: 8 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
   (10) an amino acid sequence of the heavy chain variable region of the immunoglobulin is a sequence set forth in SEQ ID NO: 6 or a variant thereof, and an amino acid sequence of the light chain variable region of the immunoglobulin is a sequence set forth in SEQ ID NO: 8 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
   (11) an amino acid sequence of the heavy chain variable region of the immunoglobulin is a sequence set forth in SEQ ID NO: 10 or a variant thereof, and an amino acid sequence of the light chain variable region of the immunoglobulin is a sequence set forth in SEQ ID NO: 12 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
   (12) an amino acid sequence of the heavy chain variable region of the immunoglobulin is a sequence set forth in SEQ ID NO: 10 or a variant thereof, and an amino acid sequence of the light chain variable region of the immunoglobulin is a sequence set forth in SEQ ID NO: 12 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
   (13) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 41 or a variant thereof, and an amino acid sequence of the light chain variable region of the single chain antibody is a sequence set forth in SEQ ID NO: 42 or a variant thereof;
   (14) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 43 or a variant thereof, and an amino acid sequence of the light chain variable region of the single chain antibody is a sequence set forth in SEQ ID NO: 44 or a variant thereof;
   (15) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 41 or a variant thereof, and an amino acid sequence of the light chain variable region of the single chain antibody is a sequence set forth in SEQ ID NO: 42 or a variant thereof;
   (16) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 43 or a variant thereof, and an amino acid sequence of the light chain variable region of the single chain antibody is a sequence set forth in SEQ ID NO: 44 or a variant thereof;
   (17) an amino acid sequence of the heavy chain variable region of the immunoglobulin is a sequence set forth in SEQ ID NO: 41 or a variant thereof, and an amino acid sequence of the light chain variable region of the immunoglobulin is a sequence set forth in SEQ ID NO: 42 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
   (18) an amino acid sequence of the heavy chain variable region of the immunoglobulin is a sequence set forth in SEQ ID NO: 43 or a variant thereof, and an amino acid sequence of the light chain variable region of the immunoglobulin is a sequence set forth in SEQ ID NO: 44 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
   (19) an amino acid sequence of the heavy chain variable region of the immunoglobulin is a sequence set forth in SEQ ID NO: 41 or a variant thereof, and an amino acid sequence of the light chain variable region of the immunoglobulin is a sequence set forth in SEQ ID NO: 42 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
      and,
   (20) an amino acid sequence of the heavy chain variable region of the immunoglobulin is a sequence set forth in SEQ ID NO: 43 or a variant thereof, and an amino acid sequence of the light chain variable region of the immunoglobulin is a sequence set forth in SEQ ID NO: 44 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof, wherein the variant has at least 60%, 70%, 80%, 81%, 82%, 83%, 84%, 85%, 86%, 87%, 88%, 89%, 90%, 91%, 92%, 93%, 94%, 95%, 96%, 97%, 98%, or at least 99% homology to the corresponding sequence.
4. The anti-CTLA4-anti-PD-1 bispecific antibody according to any one of items 1 to 3, wherein,
   an amino acid sequence of the heavy chain of the immunoglobulin is a sequence set forth in SEQ ID NO: 40 or a variant thereof, and an amino acid sequence of the light chain of the immunoglobulin is a sequence set forth in SEQ ID NO: 24 or a variant thereof, wherein a sequence of the variant has at least 60%, 70%, 80%, 81%, 82%, 83%, 84%, 85%, 86%, 87%, 88%, 89%, 90%, 91%, 92%, 93%, 94%, 95%, 96%, 97%, 98%, or at least 99% homology to the corresponding sequence.
5. The anti-CTLA4-anti-PD-1 bispecific antibody according to any one of items 1 to 4, wherein the first protein functional region is linked to the second protein functional region either directly or via a linker fragment; and/or the heavy chain variable region of the single chain antibody is linked to the light chain variable region of the single chain antibody either directly or via a linker fragment.
6. The anti-CTLA4-anti-PD-1 bispecific antibody according to item 5, wherein the linker fragment is (GGGGS)n, n being a positive integer; preferably, n is 1, 2, 3, 4, 5, or 6.
7. The anti-CTLA4-anti-PD-1 bispecific antibody according to any one of items 1 to 6, wherein the numbers of the first and second protein functional regions are each independently 1, 2, or more.
8. The anti-CTLA4-anti-PD-1 bispecific antibody according to any one of items 1 to 7, wherein the single chain antibody (preferably the heavy chain variable region) is linked to the C terminus of the heavy chain of the immunoglobulin.
9. The anti-CTLA4-anti-PD-1 bispecific antibody according to any one of items 1 to 8, wherein,
   the immunoglobulin is of human IgG1 subtype;
   wherein, according to the EU numbering system, the immunoglobulin comprises a heavy chain constant region having an A mutation selected from the following mutations:
      L234A and L235A; or
      L234A and G237A; or
      L235A and G237A; or
      L234A, L235A, and G237A; and
      according to the EU numbering system, the immunoglobulin comprises a heavy chain constant region having a B mutation selected from one or more of the following mutations:
         N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A;
         or, according to the EU numbering system, the immunoglobulin comprises a heavy chain constant region having a combination of an A mutation and a B mutation;
            or
         the heavy chain constant region of the anti-VEGFR2 monoclonal antibody or the anti-VEGF monoclonal antibody is Ig gamma-1 chain C region, ACCESSION P01857; the light chain constant region is Ig kappa chain C region, ACCESSION P01834.
10. The anti-CTLA4-anti-PD-1 bispecific antibody according to any one of items 1 to 9,
   wherein, the bispecific antibody comprises:
   a first protein functional region targeting PD-1 and
   a second protein functional region targeting CTLA4,
   the number of the first protein functional region is 1, and the number of the second protein functional region is 2;
   wherein the first protein functional region is an immunoglobulin, and the second protein functional region is a single chain antibody;
   an amino acid sequence of the heavy chain of the immunoglobulin is a sequence set forth in SEQ ID NO: 40 or a variant thereof, and an amino acid sequence of the light chain of the immunoglobulin is a sequence set forth in SEQ ID NO: 24 or a variant thereof, wherein the variant has at least 60%, 70%, 80%, 81%, 82%, 83%, 84%, 85%, 86%, 87%, 88%, 89%, 90%, 91%, 92%, 93%, 94%, 95%, 96%, 97%, 98%, or at least 99% homology to the corresponding sequence;
   an amino acid sequence of the heavy chain variable region of the single chain antibody is a sequence set forth in SEQ ID NO: 43 or a variant thereof, and an amino acid sequence of the light chain variable region of the single chain antibody is a sequence set forth in SEQ ID NO: 44 or a variant thereof, wherein the variant has at least 60%, 70%, 80%, 81%, 82%, 83%, 84%, 85%, 86%, 87%, 88%, 89%, 90%, 91%, 92%, 93%, 94%, 95%, 96%, 97%, 98%, or at least 99% homology to the corresponding sequence;
   the single chain antibody is linked to the C terminus of the heavy chain of the immunoglobulin;
   the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different;
   preferably, the first linker fragment and the second linker fragment each have an amino acid sequence independently selected from SEQ ID NO: 25 and SEQ ID NO: 26;
   preferably, amino acid sequences of the first linker fragment and second linker fragment are set forth in SEQ ID NO: 26,
   preferably, the light chain of the anti-PD-1/CTLA-4 bispecific antibody has a sequence set forth in SEQ ID NO: 72, and the heavy chain of the anti-PD-1/CTLA-4 bispecific antibody has a sequence set forth in SEQ ID NO: 70;
   preferably, the antigen-binding fragment is selected from Fab, Fab', F(ab')2, Fd, Fv, dAb, Fab/c, complementarity determining region (CDR) fragment, single chain antibody (e.g., scFv), bivalent antibody, or domain antibody.
11. A pharmaceutical composition for treating a tumor (particularly a malignant tumor), comprising an effective amount of the anti-CTLA4-anti-PD-1 bispecific antibody according to any one of items 1-10 and the component A as defined in any one of items 1-10, wherein the pharmaceutical composition is preferably in the form of a solid or liquid; preferably, the mass ratio of the anti-CTLA4-anti-PD-1 bispecific antibody and the component A is selected from (1:5)-(5:1), such as 1:5, 1:4, 1:3, 1:2, 1:1, 2:1, 3:1, 4:1, or 5:1; optionally, the pharmaceutical composition further comprises one or more chemotherapeutic drugs (preferably, the chemotherapeutic drug is an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel, albumin-bound paclitaxel, liposome paclitaxel, docetaxel, etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent).
12. Use of the anti-CTLA4-anti-PD-1 bispecific antibody according to any one of items 1-10 in preparing a medicament or a kit for preventing and/or treating a tumor (particularly a malignant tumor), wherein the medicament or the kit preferably further comprises the component A as defined in any one of items 1-10; optionally, the medicament or the kit further comprises one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel, albumin-bound paclitaxel, liposome paclitaxel, docetaxel, etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent).
13. A method for preventing and/or treating a tumor (particularly a malignant tumor), comprising administering to a subject a therapeutically effective amount of the anti-CTLA4-anti-PD-1 bispecific antibody according to any one of items 1-10 in combination with the component A as defined in any one of items 1-10, and more preferably further in combination with one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an antimetabolite, an antibiotic, a plant-based and/or hormonal drug, a platinum-based drug (e.g., cisplatin, carboplatin, and oxaliplatin), adriamycin, cyclophosphamide, paclitaxel (e.g., albumin-bound paclitaxel, liposome paclitaxel, and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent), wherein the anti-CTLA4-anti-PD-1 bispecific antibody, the component A, and the anti-tumor chemotherapeutic drug are preferably administered simultaneously or sequentially.
14. A kit for preventing and/or treating a tumor (particularly a malignant tumor), comprising (a) a first pharmaceutical composition comprising the anti-CTLA4-anti-PD-1 bispecific antibody according to any one of items 1-10 as an active ingredient; (b) a second pharmaceutical composition comprising the component A as defined in any one of items 1-10 as an active ingredient; and optionally (c) one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel (e.g., albumin-bound paclitaxel, liposome paclitaxel, and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent).
15. A unit formulation preferably used for treating a tumor (particularly a malignant tumor), wherein the unit formulation comprises 1-1000 mg (preferably 10-1000 mg, preferably 50-500 mg, 100-400 mg, 150-300 mg, 150-250 mg, or 200 mg) of the anti-CTLA4-anti-PD-1 bispecific antibody according to any one of items 1-10, 1-1000 mg (preferably 10-1000 mg, preferably 50-500 mg, 100-400 mg, 150-300 mg, 150-250 mg, or 200 mg) of the component A as defined in any one of items 1-10, and optionally one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel (e.g., albumin-bound paclitaxel, liposome paclitaxel, and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent); wherein the anti-CTLA4-anti-PD-1 bispecific antibody, the component A, and the chemotherapeutic drug are packaged separately.
16. A method for preventing or treating cancer or a tumor, wherein the method comprises administering to a subject in need thereof one or more unit formulations according to item 15; preferably, the anti-CTLA4-anti-PD-1 bispecific antibody, the component A, and the chemotherapeutic drug in the unit formulation are each administered separately.
17. A single dose unit, preferably used for treating a tumor (particularly a malignant tumor), and comprising 0.1-10000 mg (preferably 1-1000 mg, preferably 50-500 mg, 100-400 mg, 150-300 mg, 150-250 mg, 200 mg, or 100 mg) of the anti-CTLA4 anti-PD-1 bispecific antibody according to any one of items 1-10 and 0.1-10000 mg (preferably 1-1000 mg, preferably 50-500 mg, 100-400 mg, 150-300 mg, 150-250 mg, 200 mg, or 100 mg) of the component A as defined in any one of items 1-10.
18. The anti-CTLA4-anti-PD-1 bispecific antibody according to any one of items 1-10, the pharmaceutical composition according to item 11, the use according to item 12, the method according to item 13, the kit according to item 14, the unit formulation according to item 15, or the method according to item 16, wherein
   the tumor is selected from one or more of the following:
   cervical cancer (e.g., metastatic cervical cancer), lung cancer such as non-small cell lung cancer (e.g., squamous non-small cell lung cancer or non-squamous non-small cell lung cancer), esophageal cancer, esophageal squamous cancer, ileocecal adenocarcinoma, ampullate adenocarcinoma, small cell lung cancer, gastric cancer (e.g., advanced gastric cancer, gastric adenocarcinoma, or gastroesophageal junction adenocarcinoma), kidney cancer, endometrial cancer, adrenocortical carcinoma, prostate cancer, thyroid cancer, peritoneal cancer, adenocarcinoma, pancreatic cancer, glioma, head and neck cancer, bone cancer, testicular cancer, leukemia, myeloma, lymphoma, sarcoma, mesothelioma, hepatocellular carcinoma, colon cancer, biliary tract cancer, cholangiocarcinoma, rectal cancer, colon cancer, endometrial cancer, ovarian cancer (e.g., advanced ovarian cancer) or fallopian tube cancer, large cell neuroendocrine cancer, urothelial carcinoma (e.g., upper urothelial carcinoma or bladder cancer), breast cancer, triple-negative breast cancer, peripheral T-cell lymphoma, melanoma, nasopharyngeal cancer, high microsatellite instability (MSI-H) or mismatch repair deficient (dMMR) solid tumors, brain cancer (e.g., aggressive brain cancer, such as glioblastoma), ovarian cancer, squamous cell carcinoma, basal cell carcinoma, adenoma, mucinous or serous cystadenocarcinoma, leiomyosarcoma, rhabdomyosarcoma, chorioepithelioma, malignant hydatidiform mole, malignant sertoli cell-stromal cell tumor, malignant granulocytoma, and dysgerminoma.
19. The anti-CTLA4-anti-PD-1 bispecific antibody according to any one of items 1-10, the pharmaceutical composition according to item 11, the use according to item 12, the method according to item 13, the kit according to item 14, the unit formulation according to item 15, or the method according to item 16, wherein the anti-CTLA4-anti-PD-1 bispecific antibody, the component A, and/or the chemotherapeutic drug is in a form suitable for intravenous injection or intravenous drip infusion, preferably in a liquid form.
20. The method according to item 13 or 16, wherein the step of administering to the subject an effective amount of the anti-CTLA4-anti-PD-1 bispecific antibody is before or after a surgical treatment and/or before or after a radiotherapy.
21. The method according to item 13 or 16, wherein,
   a unit dose of the anti-CTLA4 anti-PD-1 bispecific antibody is 0.1-100 mg, preferably 1-10 mg per kg body weight; alternatively, a unit dose of the anti-CTLA4-anti-PD-1 bispecific antibody is 10-1000 mg, preferably 50-500 mg, 100-400 mg, 150-300 mg, 150-250 mg, 200 mg, or 100 mg in each subject;
   a unit dose of the component A is 0.1-100 mg, preferably 1-10 mg per kg body weight; alternatively, a unit dose of the component A is 10-1000 mg, preferably 50-500 mg, 100-400 mg, 150-300 mg, 150-250 mg, 200 mg, or 100 mg in each subject;
   preferably, the dose is administered from twice daily to about once every other day, or once every 3 days, 4 days, 5 days, 6 days, 10 days, 1 week, 2 weeks, or 3 weeks;
   preferably, the route of administration is intravenous drip infusion or intravenous injection.

In some embodiments, the anti-CTLA4-anti-PD-1 bispecific antibody, the anti-VEGFR2 monoclonal antibody, or the anti-VEGF monoclonal antibody is administered at a dose of 1 mg/kg, 2 mg/kg, 3 mg/kg, 5 mg/kg, 6 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, or 25 mg/kg body weight.

In some embodiments, the anti-CTLA4-anti-PD-1 bispecific antibody, the anti-VEGFR2 monoclonal antibody, or the anti-VEGF monoclonal antibody is administered at one or more flat doses capable of effectively treating the cancer. In some specific embodiments, the flat dose is in the range of about 1 mg to about 1000 mg. In some specific embodiments, the flat dose is selected from about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, or about 1000 mg.

In some embodiments, the administration of the anti-CTLA4-anti-PD-1 bispecific antibody, the anti-VEGFR2 monoclonal antibody, or the anti-VEGF monoclonal antibody is performed in cycles of 2 weeks (14 days) or 3 weeks (21 days), and preferably, the anti-PD-1-anti-CTLA4 bispecific antibody is administered intravenously on the first day (D1) of each cycle. That is, the anti-PD-1-anti-CTLA4 bispecific antibody is administered once every two weeks (q2w) or three weeks (q3w).

The present invention also provides use of the anti-CTLA4-anti-PD-1 bispecific antibody, the anti-VEGFR2 monoclonal antibody, and the anti-VEGF monoclonal antibody in preparing an article of manufacture for treating cancer comprising a container containing a fixed dose of the anti-CTLA4-anti-PD-1 bispecific antibody or the anti-VEGFR2 monoclonal antibody (or the anti-VEGF monoclonal antibody). In some specific embodiments, the container is an ampoule. The fixed dose is selected from about 10 mg, about 20 mg, about 30 mg, about 40 mg, about 50 mg, about 60 mg, about 100 mg, about 150 mg, about 200 mg, about 250 mg, about 300 mg, about 350 mg, about 400 mg, about 500 mg, or about 1000 mg. In some specific embodiments, the article of manufacture further comprises a package insert or instructions instructing the user to administer the fixed dose of the anti-CTLA4-anti-PD-1 bispecific antibody, the anti-VEGFR2 monoclonal antibody, or the anti-VEGF monoclonal antibody to a cancer patient. In some specific embodiments, the article of manufacture comprises 1 or more ampoules containing about 50 mg, 100 mg, 200 mg, 300 mg, 350 mg, 400 mg, 500 mg, or 600 mg of the anti-CTLA4-anti-PD-1 bispecific antibody, the anti-VEGFR2 monoclonal antibody, or the anti-VEGF monoclonal antibody. In some specific embodiments, the article of manufacture is packaged as per a 50 mg/5 mL/vial, 100 mg/10 mL/vial, 200 mg/10 mL/vial or 350 mg/35 mL/vial of a solution of the anti-PD-1-anti-CTLA4 bispecific antibody, the anti-VEGFR2 monoclonal antibody, or the anti-VEGF monoclonal antibody. The amount of the chemotherapeutic drug can be determined by those skilled in the art. For platinum-based chemotherapeutic drugs, in the case of cisplatin, the unit dose of cisplatin is calculated according to Calvert formula:
Cisplatin dose (mg) = area under the set curve, namely AUC (mg/mL/min) × [creatinine clearance rate (mL/min) + 25], wherein the AUC is 4-7, preferably 5;
the dose is administered once every 2-6 weeks, preferably once every 3 weeks or once every 4 weeks;
   and/or
the route of administration is intravenous drip infusion or intravenous injection.

In one or more embodiments of the present invention, the chemotherapeutic drug or the growth inhibitor is selected from an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxic agent or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof.

In one or more embodiments of the present invention, the targeted therapeutic agent is selected from a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof.

In one or more embodiments of the present invention, the antibody-drug conjugate comprises a drug selected from the group consisting of: maytansine, monomethyl auristatin E, calicheamicin, esperamicin, and a radioisotope chelating agent.

In the present invention, some expressions have the following meanings:
A "therapeutically effective amount" or "therapeutically effective dose" of a drug or therapeutic agent is any amount of a drug that, when used alone or in combination with another therapeutic agent, protects a subject from the onset of a disease or promotes disease regression as evidenced by reduction in the severity of disease symptoms, increase in the frequency and duration of disease symptom-free periods, or the prevention of damage or disability caused by the affliction of the disease. The ability of a therapeutic agent to promote disease regression can be evaluated using a variety of methods known to skilled practitioners, such as in a human subject in clinical trials, in an animal model system that predicts the efficacy in humans, or by determining the activity of the drug in an *in vitro* assay.

The "prophylactically effective amount" of a drug refers to any amount of a drug that inhibits the occurrence or recurrence of cancer when administered, alone or in combination with an antineoplastic agent, to a subject at risk of developing cancer (e.g., a subject having a premalignant condition) or a subject at risk of recurrence of cancer. In some embodiments, the prophylactically effective amount completely prevents the occurrence or recurrence of cancer. "Inhibiting" the occurrence or recurrence of cancer means reducing the possibility of the occurrence or recurrence of cancer or completely preventing the occurrence or recurrence of cancer.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

The variable regions of the light chain and the heavy chain determine the binding of the antigen: The variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3, defined by Kabat et al., see Sequences of Proteins of Immunological Interest, Fifth Edition (1991), Volumes 1-3, NIH Publication 91-3242, Bethesda Md). Given the known sequences of the heavy and light chain variable regions of an antibody, there are several methods for determining the CDR regions of the antibody, including the Kabat, IMGT, Chothia and AbM numbering systems. However, the application of all the definitions of CDRs for an antibody or its variant shall fall within the scope of the terms defined and used herein. If an amino acid sequence of the variable region of the antibody is known, those skilled in the art can generally determine a particular CDR, without relying on any experimental data beyond the sequence itself.

A "therapeutically effective amount" or "therapeutically effective dose" of a drug or therapeutic agent is any amount of a drug that, when used alone or in combination with another therapeutic agent, protects a subject from the onset of a disease or promotes disease regression as evidenced by reduction in the severity of disease symptoms, increase in the frequency and duration of disease symptom-free periods, or the prevention of damage or disability caused by the affliction of the disease. The ability of a therapeutic agent to promote disease regression can be evaluated using a variety of methods known to skilled practitioners, such as in a human subject in clinical trials, in an animal model system that predicts the efficacy in humans, or by determining the activity of the drug in an *in vitro* assay.

The "prophylactically effective amount" of a drug refers to any amount of a drug that inhibits the occurrence or recurrence of cancer when administered, alone or in combination with an antineoplastic agent, to a subject at risk of developing cancer (e.g., a subject having a premalignant condition) or a subject at risk of recurrence of cancer. In some embodiments, the prophylactically effective amount completely prevents the occurrence or recurrence of cancer. "Inhibiting" the occurrence or recurrence of cancer means reducing the possibility of the occurrence or recurrence of cancer or completely preventing the occurrence or recurrence of cancer.

A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

The term "single dose unit" means a single pharmaceutical dosage form, such as an injection, e.g. placed in an ampoule, comprising the anti-CTLA4-anti-PD-1 bispecific antibody, the anti-VEGFR2 antibody, or the anti-VEGF antibody according to the present invention to be administered to a subject at time points of a regimen, preferably per kg body weight of the subject. In a specific embodiment of the present invention, the regimen comprises, for example, administration of a single dose unit according to an administration cycle of from twice daily to about once every other day, or once every 3 days, 4 days, 5 days, 6 days, 10 days, 1 week, 2 weeks, 3 weeks, 4 weeks, 5 weeks, or 6 weeks.

In the present invention, the terms "first" (e.g., first protein functional region or first linker fragment) and "second" (e.g., second protein functional region or second linker fragment) are used for distinguishing or clarity in expression and do not carry typical sequential meanings, unless otherwise specified.

### BRIEF DESCRIPTION OF THE DRAWINGS

FIG. 1. Results of assays for the biological activity of the anti-PD-1/CTLA-4 bispecific antibody Cadonilimab in combination with the anti-VEGFR2 antibody HpL3 for promoting INF-γ secretion determined by mixed lymphocyte reaction (MLR).
FIG. 2. Results of assays for the biological activity of Cadonilimab in combination with bevacizumab for promoting PBMC activation and IL-2 secretion determined by mixed lymphocyte reaction (MLR).

### DETAILED DESCRIPTION

Embodiments of the present invention will be described in detail below with reference to examples, but those skilled in the art will appreciate that the following examples are only for illustrating the present invention, and should not be construed as limitations to the scope of the present invention. Experimental procedures without specified conditions in the examples are conducted according to conventional conditions or conditions recommended by the manufacturer. Reagents or instruments used are all commercially available conventional products if the manufacturers thereof are not specified.

In the following examples of the present invention, the isotype control antibodies used, i.e., anti-HEL, were antibodies targeting human anti-hen egg lysozyme (HEL), and the variable region sequences of these antibodies were from the study reported by Acierno et al., entitled "Affinity maturation increases the stability and plasticity of the Fv domain of anti-protein antibodies" (Acierno et al., J Mol biol., 2007; 374(1): 130-46, wherein the sequence of the heavy chain variable region is set forth in SEQ ID NO: 45, and the sequence of the light chain variable region is set forth in SEQ ID NO: 47). In the constant region fragment of hIgGIWT (i.e., anti-HEL), the heavy chain constant region (SEQ ID NO: 46) is Ig gamma-1 chain C region, ACCESSION: P01857, and the light chain constant region (SEQ ID NO: 48) is Ig kappa chain C region, ACCESSION: P01834; the anti-HEL were prepared by Akeso Biopharma, Inc.

Bevacizumab (an anti-VEGF monoclonal antibody) used in the examples was a commercial drug produced by Roche.

### Preparation Example 1: Sequence Design of Anti-CTLA4 Antibodies

The amino acid sequences and encoding nucleic acid sequences of the heavy and light chains of the anti-CTLA4 antibody 4G10 and its humanized antibodies 4G10H1L1 and 4G10H3L3 are identical to those of 4G10, 4G10H1L1 and 4G10H3L3 in Chinese Patent Publication No. CN106967172A, respectively.
(1) Heavy and light chain variable region sequences of 4G10
   nucleic acid sequence of the heavy chain variable region: (372 bp, SEQ ID NO: 1)
   its encoded amino acid sequence: (124 aa, SEQ ID NO: 2)
   nucleic acid sequence of the light chain variable region: (378 bp, SEQ ID NO: 3)
   its encoded amino acid sequence: (126 aa, SEQ ID NO: 4)
(2) Heavy and light chain variable region sequences of humanized monoclonal antibody 4G10H1L1
   nucleic acid sequence of the heavy chain variable region (4G10H1V): (345 bp, SEQ ID NO: 5)
   its encoded amino acid sequence: (115 aa, SEQ ID NO: 6)
   nucleic acid sequence of the light chain variable region (4G10L1V): (327 bp, SEQ ID NO: 7)
   its encoded amino acid sequence: (109 aa, SEQ ID NO: 8)
(3) Heavy and light chain variable region sequences of humanized monoclonal antibody 4G10H3L3
   nucleic acid sequence of the heavy chain variable region (4G10H3V): (345 bp, SEQ ID NO: 9)
   its encoded amino acid sequence of the heavy chain variable region (4G10H3V): (115 aa, SEQ ID NO: 10)
   nucleic acid sequence of the light chain variable region (4G10L3V): (327 bp, SEQ ID NO: 11)
   its encoded amino acid sequence (4G10L3V): (109 aa, SEQ ID NO: 12)

### Preparation Example 2: Sequence Design of Anti-PD-1 Antibody 14C12 and Its Humanized Antibody 14C12H1L1

The amino acid sequences and encoding nucleic acid sequences of the heavy and light chains of anti-PD-1 antibody 14C12 and its humanized antibody 14C12H1L1 are identical to those of 14C12 and 14C12H1L1 in Chinese Patent Publication No. CN106967172A, respectively.
(1) Heavy and light chain variable region sequences of 14C12
   nucleic acid sequence of the heavy chain variable region: (354 bp, SEQ ID NO: 13)
   its encoded amino acid sequence: (118 aa, SEQ ID NO: 14)
   nucleic acid sequence of the light chain variable region: (321 bp, SEQ ID NO: 15)
   its encoded amino acid sequence: (107 aa, SEQ ID NO: 16)
(2) Heavy and light chain variable region and heavy and light chain sequences of humanized monoclonal antibody 14C12H1L1
   nucleic acid sequence of the heavy chain variable region: (354 bp, SEQ ID NO: 17)
   its encoded amino acid sequence: (118 aa, SEQ ID NO: 18)
   nucleic acid sequence of the light chain variable region: (321 bp, SEQ ID NO: 19)
   its encoded amino acid sequence: (107 aa, SEQ ID NO: 20)
   DNA sequence of 14C12H1L1 heavy chain (14C12H1): (1344 bp, SEQ ID NO: 21)
   its encoded amino acid sequence: (448 aa, SEQ ID NO: 22)
   DNA sequence of 14C12H1L1 light chain (14C12L1): (642 bp, SEQ ID NO: 23)
   its encoded amino acid sequence: (214 aa, SEQ ID NO: 24)

### Preparation Example 3: Sequence Design of Bifunctional Antibodies CP001(M), CP002(M), CP003(M) and CP004(M)

The structural patterns of the bifunctional antibodies CP001(M), CPb002(M), CP003(M) and CP004(M) are in the Morrison format (IgG-scFv), i.e., C termini of two heavy chains of one IgG antibody are separately linked to the scFv fragment of another antibody via linker fragments. The components for the heavy and light chain design are shown in Table A below.

**Table A: Sequence design of CP001(M), CP002(M), CP003(M) and CP004(M)**

| Bifunctional antibody | Immunoglobulin moiety | | Linker fragment | scFv moiety |
|---|---|---|---|---|
| | Heavy chain | Light chain | | |
| CP001(M) | 14C12H1 | 14C12L1 | Linker1 | 4G10H1V(M)- Linker2 -4G10L1V(M) |
| CP002(M) | 14C12H1 | 14C12L1 | Linker2 | 4G10H1V(M)-Linker2 -4G10L1V(M) |
| CP003(M) | 14C12H1 | 14C12L1 | Linker1 | 4G10H3V(M)- Linker2 -4G10L3V(M) |
| CP004(M) | 14C12H1 | 14C12L1 | Linker2 | 4G10H3V(M)- Linker2 -4G10L3V(M) |

In Table A above:
The amino acid sequence of Linker! is (GGGGS)3 (SEQ ID NO: 25).
The amino acid sequence of Linker2 is (GGGGS)4 (SEQ ID NO: 26).

In Table A above, scFv fragments 4G10H1V(M), 4G10L1V(M), 4G10H3V(M) and 4G10L3V(M) of CP001(M), CP002(M), CP003(M) and CP004(M) antibodies comprised mutations in individual amino acids of framework regions on the basis of 4G10H1V, 4G10L1V, 4G10H3V and 4G10L3V, respectively, which effectively optimized the structure of the antibodies and improved their efficacy.
(1) 4G10H1V(M): (115 aa, mutation positions underlined in the amino acid sequence based on 4G10H1V, SEQ ID NO: 41)
(2) 4G10L1V(M): (110 aa, mutation positions underlined in the amino acid sequence based on 4G10L1V, SEQ ID NO: 42)
(3) 4G10H3V(M): (115 aa, mutation positions underlined in the amino acid sequence based on 4G10H3V, SEQ ID NO: 43)
(4) 4G10L3V(M): (110 aa, mutation positions underlined in the amino acid sequence based on 4G10L3V, SEQ ID NO: 44)

For distinguishment from the mutated antibodies hereinafter, CP004(M) is also referred to as CP004(hG1WT) in the examples of the present invention. CP004(M) described above is the "wild-type", comprising an Ig gamma-1 chain C region (ACCESSION: P01857) as the heavy chain constant region and an Ig kappa chain C region (ACCESSION: P01834) as the light chain constant region.

### Preparation Example 4: Non-Variable Region Amino Acid Mutation Design Based on Humanized Bifunctional Antibody CP004

On the basis of CP004(hG1WT) obtained in Preparation Example 3, CP004(hG1TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain.

DNA sequence of heavy chain of the immunoglobulin moiety in CP004(hG1TM): (1344 bp, mutation positions underlined, SEQ ID NO: 39) Amino acid sequence of heavy chain of the immunoglobulin moiety in CP004(hG1TM): (448 aa, mutation positions underlined, SEQ ID NO: 40) CP004(hG1TM) and CP004(hG1WT) share the same DNA sequence of light chain and the same encoded amino acid sequence. The specific sequence is shown in Preparation Example 3.

### Preparation Example 5: Preparation of Anti-PD-1/CTLA-4 Bispecific Antibody Cadonilimab

The structural pattern of the anti-PD-1/CTLA-4 bispecific antibody Cadonilimab, (i.e. CP004(hG1TM)), is in the Morrison format (IgG-scFv), i.e., C termini of two heavy chains of one immunoglobulin moiety (IgG) antibody are linked to the VH-terminus of the scFv fragment of another antibody via linker fragments, wherein the immunoglobulin moiety is based on the PD-1 antibody and the scFv fragment is based on the anti-CTLA-4 antibody, linked in between by a linker fragment.

The light chain sequence (SEQ ID NO: 72) and the heavy chain sequence (SEQ ID NO:70) of the anti-PD-1/CTLA-4 bispecific antibody Cadonilimab antibody (i.e., CP004(hG1TM)) described in this example were derived from WHO Drug Information, Proposed INN: List 124 (WHO Drug Information, 2020; 34(4): 947-949), and the preparation method is as follows.

The DNA sequence of the heavy chain-coding gene, including the constant region and the variable region, and the DNA sequence of the light chain-coding gene, including the constant region and the variable region, of the candidate monoclonal antibodies were separately cloned into pUC57simple (supplied by GenScript) vectors to obtain plasmids pUC57simple-H and pUC57simple-L, respectively.

The above plasmids were enzymatically digested (HindIII&EcoRI), and the heavy and light chains isolated by electrophoresis were separately subcloned into expression vector pcDNA3.1 (purchased from Invitrogen). The recombinant plasmids were extracted, and a combination of pcDNA3.1-H + pcDNA3.1-L was co-transfected into 293F cells. After 7 days of cell culture, the culture was centrifuged at high speed. The supernatant was concentrated, loaded onto a HiTrap MabSelect SuRe column, and purified using a protein purification liquid chromatography system (AKTA Purifier 10, GE).

The purified sample, i.e., antibody Cadonilimab, was added to both a reduced protein electrophoresis loading buffer and a non-reduced protein electrophoresis loading buffer, and then boiled for SDS-PAGE electrophoresis. The target protein of the reduced protein sample was at 70 kD and 30 kD, and the target protein of the non-reduced protein sample (single antibody) was at 200 kD. The protein conformed to the theoretical size, and thus a purified antibody was obtained.

### Preparation Example 6: Preparation of Anti-VEGFR2 Antibody HpL3

The sequence and preparation method of the antibody light chain variable region and heavy chain variable region of the anti-VEGFR2 antibody HpL3 described in this example are cited from the patent application No. CN201610573836.X, "AN ANTI-VASCULAR ENDOTHELIAL GROWTH FACTOR RECEPTOR VEGFR2 MONOCLONAL ANTIBODY AND AN ENCODING GENE AND USE THEREOF".

The amino acid sequence of the heavy chain variable region of the HpL3 is set forth in SEQ ID NO: 50, the DNA sequence of the encoding gene thereof is set forth in SEQ ID NO: 49, and the sequences of CDR1-3 of the heavy chain variable region are set forth in SEQ ID NOs: 53-55, respectively.

The amino acid sequence of the light chain variable region of the HpL3 is set forth in SEQ ID NO: 52, the DNA sequence of the encoding gene thereof is set forth in SEQ ID NO: 51, and the sequences of CDR1-3 of the light chain variable region are set forth in SEQ ID NOs: 56-58, respectively.

The HpL3 comprises an Ig gamma-1 chain C region (ACCESSION: P01857) as the heavy chain constant region and an Ig kappa chain C region (ACCESSION: P01834) as the light chain constant region.

The DNA sequence of the heavy chain-coding gene, including the constant region and the variable region, and the DNA sequence of the light chain-coding gene, including the constant region and the variable region, of the candidate monoclonal antibodies were separately cloned into pUC57simple (supplied by GenScript) vectors to obtain plasmids pUC57simple-PCABH and pUC57simple-L3, respectively.

The above plasmids were enzymatically digested (HindIII&EcoRI), and the heavy and light chains isolated by electrophoresis were separately subcloned into expression vector pcDNA3.1 (purchased from Invitrogen). The recombinant plasmids were extracted, and a combination of pcDNA3.1-PCABH + pcDNA3.1-L3 was co-transfected into 293F cells. After 7 days of cell culture, the culture was centrifuged at high speed. The supernatant was concentrated, loaded onto a HiTrap MabSelect SuRe column, and purified using a protein purification liquid chromatography system (AKTA Purifier 10, GE).

The purified sample, i.e., antibody HpL3, was added to both a reduced protein electrophoresis loading buffer and a non-reduced protein electrophoresis loading buffer, and then boiled for SDS-PAGE electrophoresis. The target protein of the reduced protein sample was at 45 kD and 30 kD, and the target protein of the non-reduced protein sample (single antibody) was at 150 kD.

The protein conformed to the theoretical size, and thus a purified monoclonal antibody was obtained.

Example 1: Biological Activity of Anti-PD-1/CTLA-4 Bispecific Antibody Cadonilimab in Combination with Anti-VEGFR2 Antibody HpL3 for Promoting INF-γ Secretion Determined by Mixed Lymphocyte Reaction (MLR)

### Preparation of Raji-PDL1 cells:

Human PD-L1-overexpressing lentiviral vector plenti6.3/V5-PDL1FL-BSD infected Raji cells after virus packaging, and a drug-resistant cell line Raji-PDL1 was obtained after BSD (Blasticidin, 10 ug/mL) drug screening. The plenti6.3/V5-PDL1FL-BSD was obtained by synthesizing the gene PDL1FL (PDL1, Genebank ID: NP_054862.1) from the supplier Nanjing Genscript, and then ligating it to plenti6.3/V5-BSD (Invitrogen Cat. No: k531520) by enzyme digestion.

Two days prior to the experiment, PBMCs (isolated from normal human peripheral blood) were thawed and cultured in a complete medium at 37 °C in a 5% carbon dioxide incubator; after 2 h, when the PBMC state was recovered, SEB (with a final concentration of 0.5 µg/mL) (Toxin technology, Cat. No: BT202) was added for stimulation for two days; on the day of the experiment, Raji-PDL1 cells were collected, centrifuged, resuspended (in 1640 + 10% FBS), counted and adjusted to a density of 2 × 10⁶/mL, MMC (Mito-mycin C, with a final concentration of 2 µg/mL, manufacturer: Stressmarq, Cat. No: SIH-246) was added, and the mixture was treated at 37 °C for 1 h in a 5% carbon dioxide incubator; PBMCs after two days of SEB stimulation and Raji-PDL1 cells after MMC treatment were collected and washed twice by 1640 basic medium, the cells were resuspended by a complete medium and counted, and the cells were inoculated into a 96-well U-shaped plate (3799) according to 1 × 10⁵/well of PBMCs and 1 × 10⁵/well of Raji-PDL1 (50 µL/well each); drugs were added according to the experimental design, blank control and negative control were set, and the cells were cultured for 3 days (the final volume of the system was 200 µL); after 3 days, the cells were centrifuged at 250× g for 5 min (Beckman centrifuge), cell supernatants were collected, and IFN-γ content was detected using Dakewe kit.

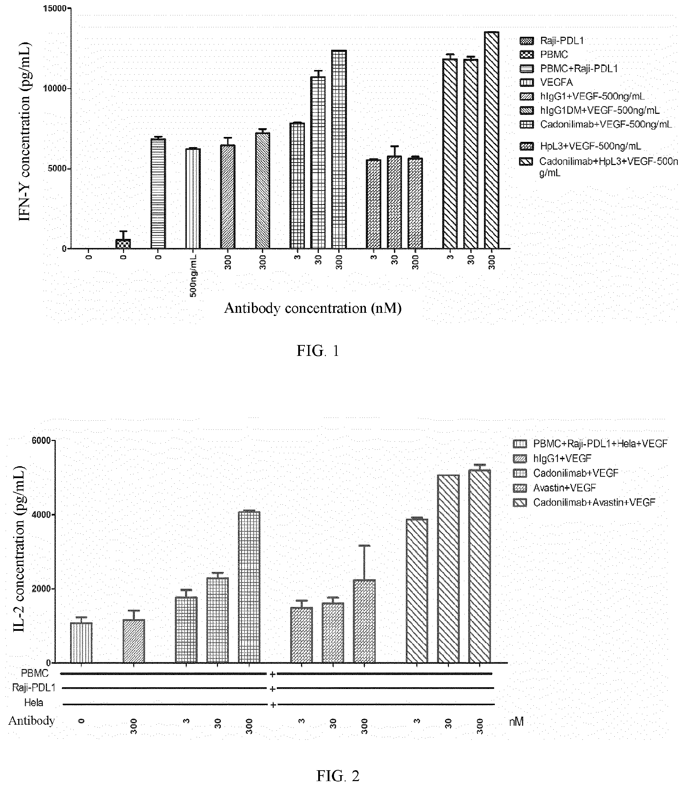

As shown in FIG. 1, in a PBMC and Raji-PDL1 cell mixed culture system, the addition of Cadonilimab and HpL3 antibody can both significantly induce secretion of IFN-γ in PBMCs in a significant dose-dependent manner. In terms of the level of promoting IFN-γ secretion activity, Cadonilimab in combination with HpL3 has better IFN-γ secretion promotion potential compared with the Cadonilimab and HpL3 antibody alone.

### Example 2: Biological Activity of Cadonilimab in Combination with Bevacizumab for Promoting PBMC Activation and IL-2 Secretion Determined by Mixed Lymphocyte Reaction (MLR)

Two days prior to the experiment, PBMCs (isolated from voluntary donation peripheral blood after informed consent from healthy volunteers) were thawed and cultured in a complete medium at 37 °C in a 5% carbon dioxide incubator; after 2 h, when the PBMC state was recovered, SEB (with a final concentration of 0.5 µg/mL) (Toxin technology, Cat. No: BT202) was added for stimulation for two days; on the day of the experiment, Raji-PDL1 (constructed by Akeso Biopharma, Inc.) cells were collected, centrifuged, resuspended (in 1640 + 10% FBS), counted and adjusted to a density of 2 × 10⁶/mL, MMC (Mito-mycin C, with a final concentration of 2 µg/mL) (mitomycin C, manufacturer: Stressmarq, Cat. No: SIH-246) was added, and the mixture was treated at 37 °C for 1 h in a 5% carbon dioxide incubator; PBMCs after two days of SEB stimulation and Raji-PDL1 cells after MMC treatment were collected and washed twice by 1640 basic medium, the cells were resuspended by an analysis medium and counted, and the cells were inoculated into a 96-well U-shaped plate (3799) according to 1 × 10⁵/well of PBMCs and 1 × 10⁵/well of Raji-PDL1 (40 µL/well each); Hela cells (purchased from Chinese academy of sciences) were digested conventionally and inoculated into a 96-well U-shaped plate (3799) according to 2 × 10⁴/well (40 µL/well); drugs were added according to the experimental design, negative control and isotype control were set, and the cells were cultured for 3 days (the final volume of the system was 200 µL); after 3 days, the cells were centrifuged at 250× g for 5 min (Beckman centrifuge), cell supernatants were collected, and IL-2 content was detected using Dakewe kit.

As shown in FIG. 2, in a PBMC, Raji-PDL1, and Hela (cervical cancer) cell, and VEGF (Batch No: 20180126-1, produced by Akeso Biopharma, Inc.) mixed culture system, the addition of Cadonilimab and bevacizumab can both significantly induce secretion of IL-2 in PBMCs in a significant dose-dependent manner. In terms of the level of promoting IL-2 secretion activity, Cadonilimab in combination with bevacizumab has better IL-2 secretion promotion potential compared with the Cadonilimab and bevacizumab alone.

The amino acid sequence of the heavy chain variable region of bevacizumab is set forth in SEQ ID NO: 60, the DNA sequence of the encoding gene thereof is set forth in SEQ ID NO: 59, and the sequences of CDR1-3 of the heavy chain variable region are set forth in SEQ ID NOs: 63-65, respectively. The amino acid sequence of the light chain variable region of bevacizumab is set forth in SEQ ID NO: 62, the DNA sequence of the encoding gene thereof is set forth in SEQ ID NO: 61, and the sequences of CDR1-3 of the light chain variable region are set forth in SEQ ID NOs: 66-68, respectively.

### SEQUENCE LISTING

SEQ ID NO: 1: nucleic acid sequence of heavy chain variable region of 4G10 
caggtcaagctgcaggagtctggacctgagctggtgaagcctggagcttcaatgaagatatcctgcaaggcttct
ggttactcattcactggctacaccatgaactgggtgaagcagagccatggaaagaaccttgaatggattggactt
attaatccttacaataatattactaactacaaccagaagttcatgggcaaggccacatttactgtagacaagtcatcc
agcacagcctacatggaactcctcagactgacatctgaagactctggagtctatttctgtgcaagactcgactata
ggtcttattggggccaagggactctggtcactgtctctgcagccaaaacgacacccccatctgtct at
SEQ ID NO: 2: amino acid sequence of heavy chain variable region of 4G10 
QVKLQESGPELVKPGASMKISCKASGYSFTGYTMNWVKQSHGKNLEW
IGLINPYNNITNYNQKFMGKATFTVDKSSSTAYMELLRLTSEDSGVYFC
ARLDYRSYWGQGTLVTVSAAKTTPPSVY
SEQ ID NO: 3: nucleic acid sequence of light chain variable region of 4G10 
caggctgttgtgactcaggaatctgcactcaccacatcacctggtgaaacagtcacactcacttgtcgctcaagta
ctggggctgttacaactagtaactttgccaactgggtccaagaaaaaccagatcatttattcactagtctaataggt
ggtaccaacaaccgagctccaggtgttcctgccagattctcaggctccctgattggagacaaggctgccctcacc
atcacaggggcacagactgaggatgaggcaatatatttctgtgctctatggtacagcaaccattgggtgttcggtg
gaggaaccaaactgactgtcctaggccagcccaagtcttcgccatcagtcaccctgtttcaagggcaattctgc
SEQ ID NO: 4: amino acid sequence of light chain variable region of 4G10 
QAVVTQESALTTSPGETVTLTCRSSTGAVTTSNFANWVQEKPDHLFTSLI
GGTNNRAPGVPARFSGSLIGDKAALTITGAQTEDEAIYFCALWYSNHW
VFGGGTKLTVLGQPKSSPSVTLFQGQFC
SEQ ID NO: 5: nucleic acid sequence of heavy chain variable region of 4G10H1L1 
caggtgcagctggtggagtctggggccgagctggtgaagcccggcgcctccatgaagatctcttgcaaggcca
gcggatacagtttcactggctataccatgaactgggtcaaacaggctccaggacagggactggagtggatcgg
 
gctgattaatccttacaacaacatcaccaactacaaccagaagttcatgggaaaagcaacctttacagtggacaa
gagcatttccacagcctacatggaactgagccggctgacttcagacgatagcggggtctatttttgtgcaaggctg
gattatcgctcttactgggggcagggaactctggtcactgtctccgct
SEQ ID NO: 6: amino acid sequence of heavy chain variable region of 4G10H1L1 
QVQLVESGAELVKPGASMKISCKASGYSFTGYTMNWVKQAPGQGLE
WIGLINPYNNITNYNQKFMGKATFTVDKSISTAYMELSRLTSDDSGVYF
CARLDYRSYWGQGTLVTVSA
SEQ ID NO: 7: nucleic acid sequence of light chain variable region of 4G10H1L1 
caggctgtcgtcactcaggaaccttcactgactgtgagcccaggaggaactgtcaccctgacatgcggaagctc
caccggagcagtgaccacatccaacttcgccaattgggtccaggaaaagccaggccaggcatttcgatccctg
atcggaggcacaaacaatcgggcttcttgggtgcccgcaagattctcaggaagcctgctggggggaaaagccg
ctctgaccattagtggcgctcagcctgaggacgaagccgagtacttctgcgctctgtggtatagcaaccactggg
tgtttggcgggggaacaaagctgactgtgctg
SEQ ID NO: 8: amino acid sequence of light chain variable region of 4G10H1L1 
QAVVTQEPSLTVSPGGTVTLTCGSSTGAVTTSNFANWVQEKPGQAFRSL
IGGTNNRASWVPARFSGSLLGGKAALTISGAQPEDEAEYFCALWYSNH
WVFGGGTKLTVL
SEQ ID NO: 9: nucleic acid sequence of heavy chain variable region of 4G10H3L3 
caggtgcagctggtcgagtctggggccgaagtgaagaaacccggcgcctcagtgaaggtcagctgcaaggcc
agcgggtacagtttcactggatataccatgaactgggtccgacaggcccctggccaggggctggagtggatcg
gcctgattaacccttacaacaacatcactaactacgcacagaagttccaggggagagtgacctttacagtggaca
 
ccagcatttccacagcctac
atggaactgtcccggctgagatctgacgatacaggcgtgtacttctgcgctaggctggattaccgcagctattgg
ggacagggcacactggtgactgtcagcgca
SEQ ID NO: 10: amino acid sequence of heavy chain variable region of 4G10H3L3 
QVQLVESGAEVKKPGASVKVSCKASGYSFTGYTMNWVRQAPGQGLE
WIGLINPYNNITNYAQKFQGRVTFTVDTSISTAYMELSRLRSDDTGVYF
CARLDYRSYWGQGTLVTVSA
SEQ ID NO: 11: nucleic acid sequence of light chain variable region of 4G10H3L3 
caggctgtcgtcactcaggaaccttcactgaccgtgtctcctggcgggactgtcaccctgacatgcggcagctcc
acaggggccgtgaccacaagtaacttcccaaattgggtccagcagaagccaggacaggctccccggagtctg
atcggaggcaccaacaacaaggccagctggacacccgcacggttcagcggcagcctgctgggcggcaagg
ccgctctgacaattagcggagcccagcctgaggacgaagccgagtactattgcgctctgtggtactccaaccac
tgggtgttcggcggcggcaccaagctgactgtgctg
SEQ ID NO: 12: amino acid sequence of light chain variable region of 4G10H3L3 
QAVVTQEPSLTVSPGGTVTLTCGSSTGAVTTSNFPNWVQQKPGQAPRSL
IGGTNNKASWTPARFSGSLLGGKAALTISGAQPEDEAEYYCALWYSNH
WVFGGGTKLTVL
SEQ ID NO: 13: nucleic acid sequence of heavy chain variable region of 14C12 
gaggtcaaactggtggagagcggcggcgggctggtgaagcccggcgggtcactgaaactgagctgcgccgc
ttccggcttcgcctttagctcctacgacatgtcatgggtgaggcagacccctgagaagcgcctggaatgggtcgc
tactatcagcggaggcgggcgatacacctactatcctgactctgtcaaagggagattcacaattagtcgggataa
 
cgccagaaatactctgtatctgcagatgtctagtctgcggtccgaggatacagctctgtactattgtgcaaaccggt
acggcgaagcatggtttgcctattggggacagggcaccctggtgacagtctctgcc
SEQ ID NO: 14: amino acid sequence of heavy chain variable region of 14C12 
EVKLVESGGGLVKPGGSLKLSCAASGFAFSSYDMSWVRQTPEKRLEWV
ATISGGGRYTYYPDSVKGRFTISRDNARNTLYLQMSSLRSEDTALYYCA
NRYGEAWFAYWGQGTLVTVSA
SEQ ID NO: 15: nucleic acid sequence of light chain variable region of 14C12 
gacattaagatgacacagtccccttcctcaatgtacgctagcctgggcgagcgagtgaccttcacatgcaaagca
tcccaggacatcaacacatacctgtcttggtttcagcagaagccaggcaaaagccccaagaccctgatctaccg
ggccaatagactggtggacggggtccccagcagattctccggatctggcagtgggcaggattactccctgacc
atcagctccctggagtatgaagacatgggcatctactattgcctgcagtatgatgagttccctctgacctttggagc
aggcacaaaactggaactgaag
SEQ ID NO: 16: amino acid sequence of light chain variable region of 14C12 
DIKMTQSPSSMYASLGERVTFTCKASQDINTYLSWFQQKPGKSPKTLIY
RANRLVDGVPSRFSGSGSGQDYSLTISSLEYEDMGIYYCLQYDEFPLTF
GAGTKLELK
SEQ ID NO: 17: nucleic acid sequence of heavy chain variable region of 14C12H1L1 
gaagtgcagctggtcgagtctgggggagggctggtgcagcccggcgggtcactgcgactgagctgcgcagct
tccggattcgcctttagctcctacgacatgtcctgggtgcgacaggcaccaggaaagggactggattgggtcgct
actatctcaggaggcgggagatacacctactatcctgacagcgtcaagggccggttcacaatctctagagataac
agtaagaacaatctgtatctgcagatgaacagcctgagggctgaggacaccgcactgtactattgtgccaaccg
ctacggggaagcatggtttgcctattgggggcagggaaccctggtgacagtctctagt
SEQ ID NO: 18: amino acid sequence of heavy chain variable region of 14C12H1L1 
EVQLVESGGGLVQPGGSLRLSCAASGFAFSSYDMSWVRQAPGKGLDW
VATISGGGRYTYYPDSVKGRFTISRDNSKNNLYLQMNSLRAEDTALYYC
ANRYGEAWFAYWGQGTLVTVSS
SEQ ID NO: 19: nucleic acid sequence of light chain variable region of 14C12H1L1 
gacattcagatgactcagagcccctcctccatgtccgcctctgtgggcgacagggtcaccttcacatgccgcgct
agtcaggatatcaacacctacctgagctggtttcagcagaagccagggaaaagccccaagacactgatctaccg
ggctaatagactggtgtctggagtcccaagtcggttcagtggctcagggagcggacaggactacactctgacca
tcagctccctgcagcctgaggacatggcaacctactattgcctgcagtatgatgagttcccactgacctttggcgc
cgggacaaaactggagctgaag
SEQ ID NO: 20: amino acid sequence of light chain variable region of 14C12H1L1 
DIQMTQSPSSMSASVGDRVTFTCRASQDINTYLSWFQQKPGKSPKTLIY
RANRLVSGVPSRFSGSGSGQDYTLTISSLQPEDMATYYCLQYDEFPLTF
GAGTKLELK
SEQ ID NO: 21: DNA sequence of 14C12H1L1 heavy chain (14C12H1) (1344 bp) 
GAAGTGCAGCTGGTCGAGTCTGGGGGAGGGCTGGTGCAGCCCGGC
GGGTCACTGCGACTGAGCTGCGCAGCTTCCGGATTCGCCTTTAGCTC
CTACGACATGTCCTGGGTGCGACAGGCACCAGGAAAGGGACTGGAT
TGGGTCGCTACTATCTCAGGAGGCGGGAGATACACCTACTATCCTGA
CAGCGTCAAGGGCCGGTTCACAATCTCTAGAGATAACAGTAAGAAC
AATCTGTATCTGCAGATGAACAGCCTGAGGGCTGAGGACACCGCAC
TGTACTATTGTGCCAACCGCTACGGGGAAGCATGGTTTGCCTATTGG
GGGCAGGGAACCCTGGTGACAGTCTCTAGTGCCAGCACCAAAGGAC
 
CTAGCGTGTTTCCTCTCGCCCCCTCCTCCAAAAGCACCAGCGGAGGA
ACCGCTGCTCTCGGATGTCTGGTGAAGGACTACTTCCCTGAACCCGT
CACCGTGAGCTGGAATAGCGGCGCTCTGACAAGCGGAGTCCATACAT
TCCCTGCTGTGCTGCAAAGCAGCGGACTCTATTCCCTGTCCAGCGTC
GTCACAGTGCCCAGCAGCAGCCTGGGCACCCAGACCTACATCTGTA
ACGTCAACCACAAGCCCTCCAACACCAAGGTGGACAAGAAAGTGG
AGCCCAAATCCTGCGACAAGACACACACCTGTCCCCCCTGTCCTGCT
CCCGAACTCCTCGGAGGCCCTAGCGTCTTCCTCTTTCCTCCCAAACC
CAAGGACACCCTCATGATCAGCAGAACCCCTGAAGTCACCTGTGTC
GTCGTGGATGTCAGCCATGAGGACCCCGAGGTGAAATTCAACTGGTA
TGTCGATGGCGTCGAGGTGCACAACGCCAAAACCAAGCCCAGGGAG
GAACAGTACAACTCCACCTACAGGGTGGTGTCCGTGCTGACAGTCC
TCCACCAGGACTGGCTGAACGGCAAGGAGTACAAGTGCAAGGTGTC
CAACAAGGCTCTCCCTGCCCCCATTGAGAAGACCATCAGCAAGGCC
AAAGGCCAACCCAGGGAGCCCCAGGTCTATACACTGCCTCCCTCCA
GGGACGAACTCACCAAGAACCAGGTGTCCCTGACCTGCCTGGTCAA
GGGCTTTTATCCCAGCGACATCGCCGTCGAGTGGGAGTCCAACGGA
CAGCCCGAGAATAACTACAAGACCACCCCTCCTGTCCTCGACTCCGA
CGGCTCCTTCTTCCTGTACAGCAAGCTGACCGTGGACAAAAGCAGG
TGGCAGCAGGGAAACGTGTTCTCCTGCAGCGTGATGCACGAAGCCC
TCCACAACCACTACACCCAGAAAAGCCTGTCCCTGAGCCCCGGCAA
A
SEQ ID NO: 22: encoded amino acid sequence of 14C12H1L1 heavy chain (14C12H1) (448 aa) 
EVQLVESGGGLVQPGGSLRLSCAASGFAFSSYDMSWVRQAPGKGLDW
VATISGGGRYTYYPDSVKGRFTISRDNSKNNLYLQMNSLRAEDTALYYC
ANRYGEAWFAYWGQGTLVTVSSASTKGPSVFPLAPSSKSTSGGTAALG
CLVKDYFPEPVTVSWNSGALTSGVHTFPAVLQSSGLYSLSSVVTVPSSSL
 
GTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHN
HYTQKSLSLSPGK
SEQ ID NO: 23: DNA sequence of 14C12H1L1 light chain (14C12L1) (642 bp) 
GACATTCAGATGACTCAGAGCCCCTCCTCCATGTCCGCCTCTGTGGG
CGACAGGGTCACCTTCACATGCCGCGCTAGTCAGGATATCAACACCT
ACCTGAGCTGGTTTCAGCAGAAGCCAGGGAAAAGCCCCAAGACACT
GATCTACCGGGCTAATAGACTGGTGTCTGGAGTCCCAAGTCGGTTCA
GTGGCTCAGGGAGCGGACAGGACTACACTCTGACCATCAGCTCCCT
GCAGCCTGAGGACATGGCAACCTACTATTGCCTGCAGTATGATGAGT
TCCCACTGACCTTTGGCGCCGGGACAAAACTGGAGCTGAAGCGAAC
TGTGGCCGCTCCCTCCGTCTTCATTTTTCCCCCTTCTGACGAACAGCT
GAAATCAGGCACAGCCAGCGTGGTCTGTCTGCTGAACAATTTCTACC
CTAGAGAGGCAAAAGTGCAGTGGAAGGTCGATAACGCCCTGCAGTC
CGGCAACAGCCAGGAGAGTGTGACTGAACAGGACTCAAAAGATAG
CACCTATTCCCTGTCTAGTACACTGACTCTGTCCAAGGCTGATTACGA
GAAGCACAAAGTGTATGCATGCGAAGTGACACATCAGGGACTGTCA
AGCCCCGTGACTAAGTCTTTTAACCGGGGCGAATGT
SEQ ID NO: 24: encoded amino acid sequence of 14C12H1L1 light chain (14C12L1) (214 aa) 
DIQMTQSPSSMSASVGDRVTFTCRASQDINTYLSWFQQKPGKSPKTLIY
RANRLVSGVPSRFSGSGSGQDYTLTISSLQPEDMATYYCLQYDEFPLTF
GAGTKLELKRTVAAPSVFIFPPSDEQLKSGTASVVCLLNNFYPREAKVQ
 
WKVDNALQSGNSQESVTEQDSKDSTYSLSSTLTLSKADYEKHKVYACE
VTHQGLSSPVTKSFNRGEC
SEQ ID NO: 25: amino acid sequence of Linker!
   GGGGSGGGGSGGGGS
SEQ ID NO: 26: amino acid sequence of Linker2
   GGGGSGGGGSGGGGSGGGGS
SEQ ID NO: 27: HCDR1 of 14C12: GFAFSSYD
SEQ ID NO: 28: HCDR2 of 14C12: ISGGGRYT
SEQ ID NO: 29: HCDR3 of 14C12: ANRYGEAWFAY
SEQ ID NO: 30: LCDR1 of 14C12: QDINTY
SEQ ID NO: 31: LCDR2 of 14C12: RAN
SEQ ID NO: 32: LCDR3 of 14C12: LQYDEFPLT
SEQ ID NO: 33: HCDR1 of 4G10: GYSFTGYT
SEQ ID NO: 34: HCDR2 of 4G10: INPYNNIT
SEQ ID NO: 35: HCDR3 of 4G10: ARLDYRSY
SEQ ID NO: 36: LCDR1 of4G10: TGAVTTSNF
SEQ ID NO: 37: LCDR2 of 4G10: GTN
SEQ ID NO: 38: LCDR3 of 4G10: ALWYSNHWV
SEQ ID NO: 39: DNA sequence of heavy chain of immunoglobulin moiety of CP004(hG1TM), mutation positions underlined 
GAAGTGCAGCTGGTCGAGTCTGGGGGAGGGCTGGTGCAGCCCGGC
GGGTCACTGCGACTGAGCTGCGCAGCTTCCGGATTCGCCTTTAGCTC
CTACGACATGTCCTGGGTGCGACAGGCACCAGGAAAGGGACTGGAT
TGGGTCGCTACTATCTCAGGAGGCGGGAGATACACCTACTATCCTGA
CAGCGTCAAGGGCCGGTTCACAATCTCTAGAGATAACAGTAAGAAC
AATCTGTATCTGCAGATGAACAGCCTGAGGGCTGAGGACACCGCAC
TGTACTATTGTGCCAACCGCTACGGGGAAGCATGGTTTGCCTATTGG
GGGCAGGGAACCCTGGTGACAGTCTCTAGTGCCAGCACCAAAGGGC
CCAGCGTGTTTCCTCTCGCCCCCTCCTCCAAAAGCACCAGCGGAGG
AACCGCTGCTCTCGGATGTCTGGTGAAGGACTACTTCCCTGAACCCG
TCACCGTGAGCTGGAATAGCGGCGCTCTGACAAGCGGAGTCCATACA
TTCCCTGCTGTGCTGCAAAGCAGCGGACTCTATTCCCTGTCCAGCGT
CGTCACAGTGCCCAGCAGCAGCCTGGGCACCCAGACCTACATCTGT
AACGTCAACCACAAGCCCTCCAACACCAAGGTGGACAAGAAAGTG
GAGCCCAAATCCTGCGACAAGACACACACCTGTCCCCCCTGTCCTG
CTCCCGAAGCTGCTGGAGCCCCTAGCGTCTTCCTCTTTCCTCCCAAA
CCCAAGGACACCCTCATGATCAGCAGAACCCCTGAAGTCACCTGTG
TCGTCGTGGATGTCAGCCATGAGGACCCCGAGGTGAAATTCAACTG
GTATGTCGATGGCGTCGAGGTGCACAACGCCAAAACCAAGCCCAGG
GAGGAACAGTACAACTCCACCTACAGGGTGGTGTCCGTGCTGACAG
TCCTCCACCAGGACTGGCTGAACGGCAAGGAGTACAAGTGCAAGGT
GTCCAACAAGGCTCTCCCTGCCCCCATTGAGAAGACCATCAGCAAG
GCCAAAGGCCAACCCAGGGAGCCCCAGGTCTATACACTGCCTCCCT
 
CCAGGGACGAACTCACCAAGAACCAGGTGTCCCTGACCTGCCTGGT
CAAGGGCTTTTATCCCAGCGACATCGCCGTCGAGTGGGAGTCCAAC
GGACAGCCCGAGAATAACTACAAGACCACCCCTCCTGTCCTCGACTC
CGACGGCTCCTTCTTCCTGTACAGCAAGCTGACCGTGGACAAAAGC
AGGTGGCAGCAGGGAAACGTGTTCTCCTGCAGCGTGATGCACGAAG
CCCTCCACAACCACTACACCCAGAAAAGCCTGTCCCTGAGCCCCGG
CAAA
SEQ ID NO: 40: amino acid sequence of heavy chain of immunoglobulin moiety in CP004(hG1TM), mutation positions underlined 
EVQLVESGGGLVQPGGSLRLSCAASGFAFSSYDMSWVRQAPGKGLDW
VATISGGGRYTYYPDSVKGRFTISRDNSKNNLYLQMNSLRAEDTALYYC
ANRYGEAWFAYWGQGTLVTVSSASTKGPSVFPLAPSSKSTSGGTAALG
CLVKDYFPEPVTVSWNSGALTSGVHTFPAVLQSSGLYSLSSVVTVPSSSL
GTQTYICNVNHKPSNTKVDKKVEPKSCDKTHTCPPCPAPEAAGAPSVF
LFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALH
NHYTQKSLSLSPGK
SEQ ID NO: 41: amino acid sequence of 4G10H1V(M), mutation positions underlined 
QVQLVESGAELVKPGASMKISCKASGYSFTGYTMNWVKQAPGQCLEW
IGLINPYNNITNYNQKFMGKATFTVDKSISTAYMELSRLTSDDSGVYFC
ARLDYRSYWGQGTLVTVSA
SEQ ID NO: 42: amino acid sequence of 4G10L1V(M), mutation positions underlined 
QAVVTQEPSLTVSPGGTVTLTCGSSTGAVTTSNFANWVQEKPGQAFRSL
IGGTNNRASWVPARFSGSLLGGKAALTISGAQPEDEAEYFCALWYSNH
WVFGCGTKLTVLR
SEQ ID NO: 43: amino acid sequence of 4G10H3V(M), mutation positions underlined 
QVQLVESGAEVKKPGASVKVSCKASGYSFTGYTMNWVRQAPGQCLE
WIGLINPYNNITNYAQKFQGRVTFTVDTSISTAYMELSRLRSDDTGVYF
CARLDYRSYWGQGTLVTVSA
SEQ ID NO: 44: amino acid sequence of 4G10L3V(M), mutation positions underlined 
QAVVTQEPSLTVSPGGTVTLTCGSSTGAVTTSNFPNWVQQKPGQAPRSL
IGGTNNKASWTPARFSGSLLGGKAALTISGAQPEDEAEYYCALWYSNH
WVFGCGTKLTVLR
SEQ ID NO: 45: sequence of heavy chain variable region of hIgG 
EVQLEQSGAELMKPGASVKISCKATGYTFTTYWIEWIKQRPGHSLEWI
GEILPGSDSTYYNEKVKGKVTFTADASSNTAYMQLSSLTSEDSAVYYCA
RGDGFYVYWGQGTTLTVSS
SEQ ID NO: 46 sequence of heavy chain constant region of hIgG 
ASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRV
ESKYGPPCPPCPAPEFLGGPSVFLFPPKPKDTLMISRTPEVTCVVVDVSQ
EDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTV
DKSRWQEGNVFSCSVMHEALHNHYTQKSLSLSLGK
SEQ ID NO: 47 sequence of light chain variable region of hIgG 
DIELTQSPATLSVTPGDSVSLSCRASQSISNNLHWYQQKSHESPRLLIKY
TSQSMSGIPSRFSGSGSGTDFTLSINSVETEDFGVYFCQQSGSWPRTFGG
GTKLDIK
SEQ ID NO: 48 sequence of light chain constant region of hIgG
SEQ ID NO: 49 nucleic acid sequence of heavy chain variable region of HpL3 (VEGFR2) 
GAAGTGCAGCTGGTGCAGAGCGGCGGGGGACTGGTGAAGCCCGGC
GGGTCCCTGCGACTGTCTTGCGCCGCTAGTGGCTTCACCTTCAGCTC
CTACTCTATGAACTGGGTGAGACAGGCCCCTGGAAAAGGCCTGGAG
TGGGTCTCTAGTATCTCAAGCTCCTCTAGTTACATCTACTATGCCGAC
AGCGTGAAGGGGCGGTTCACCATCTCAAGAGATAACGCTAAAAATA
GTCTGTATCTGCAGATGAACAGCCTGAGGGCAGAAGACACTGCCGT
GTACTATTGTGCTCGCGTCACCGACGCATTTGATATTTGGGGGCAGG
GAACCATGGTGACAGTCTCAAGC (Note: the underlined parts represent
CDR sequences)
SEQ ID NO: 50 amino acid sequence of heavy chain variable region of HpL3 


 (Note: the underlined parts represent CDR
sequences)
SEQ ID NO: 51 nucleic acid sequence of light chain variable region of HpL3 
GACATTCAGATGACTCAGAGCCCTTCTTCAGTGTCCGCCTCTATCGG
CGACCGGGTCACCATTACATGCAGAGCTTCCCAGGGCCTCGATAACT
GGCTGGGGTGGTACCAGCAGAAGCCTGGGAAAGCCCCAAAGCTGCT
GATCTACGACGCTTCCAATCTGGATACCGGAGTGCCATCTCGATTCAG
TGGCTCAGGGAGCGGAACATACTTTACTCTGACCATCAGCTCCCTGC
AGGCTGAGGACTTCGCAGTGTATTTTTGCCAGCAGGCAAAGGCCTTC
CCTCCCACCTTTGGCGGGGGAACAAAAGTGGACATCAAG (Note: the
underlined parts represent CDR sequences)
SEQ ID NO: 52 amino acid sequence of light chain variable region of HpL3 
DIQMTQSPSSVSASIGDRVTITCRASQGLDNWLGWYQQKPGKAPKLLI
YDASNLDTGVPSRFSGSGSGTYFTLTISSLQAEDFAVYFCQQAKAFPPTF
GGGTKVDIK (Note: the underlined parts represent CDR sequences)
HCDR1-HCDR3 of the heavy chain variable region of HpL3
HCDR1: GFTFSSYS (SEQ ID NO: 53)
HCDR2: ISSSSSYI (SEQ ID NO: 54)
HCDR3: ARVTDAFDI (SEQ ID NO: 55)
LCDR1-LCDR3 of the light chain variable region of HpL3
LCDR1: QGLDNW (SEQ ID NO: 56)
LCDR2: DAS (SEQ ID NO: 57)
LCDR3: QQAKAFPPT (SEQ ID NO: 58)
SEQ ID NO: 59 nucleic acid sequence of heavy chain variable region of bevacizumab (VEGF) 
GAGGTGCAGCTGGTCGAGTCCGGGGGGGGGCTGGTGCAGCCAGGC
GGGTCTCTGAGGCTGAGTTGCGCCGCTTCAGGGTACACCTTCACAA
ACTATGGAATGAATTGGGTGCGCCAGGCACCAGGAAAGGGACTGGA
GTGGGTCGGCTGGATCAACACTTACACCGGGGAACCTACCTATGCAG
 
CCGACTTTAAGCGGCGGTTCACCTTCAGCCTGGATACAAGCAAATCC
ACTGCCTACCTGCAGATGAACAGCCTGCGAGCTGAGGACACCGCAG
TCTACTATTGTGCTAAATATCCCCACTACTATGGGAGCAGCCATTGGTA
TTTTGACGTGTGGGGGCAGGGGACTCTGGTGACAGTGAGCAGC
SEQ ID NO: 60 amino acid sequence of heavy chain variable region of bevacizumab
SEQ ID NO: 61 nucleic acid sequence of light chain variable region of bevacizumab 
GATATTCAGATGACTCAGAGCCCCTCCTCCCTGTCCGCCTCTGTGGG
CGACAGGGTCACCATCACATGCAGTGCTTCACAGGATATTTCCAACT
ACCTGAATTGGTATCAGCAGAAGCCAGGAAAAGCACCCAAGGTGCT
GATCTACTTCACTAGCTCCCTGCACTCAGGAGTGCCAAGCCGGTTCA
GCGGATCCGGATCTGGAACCGACTTTACTCTGACCATTTCTAGTCTGC
AGCCTGAGGATTTCGCTACATACTATTGCCAGCAGTATTCTACCGTGC
CATGGACATTTGGCCAGGGGACTAAAGTCGAGATCAAG
SEQ ID NO: 62 amino acid sequence of light chain variable region of bevacizumab

The amino acid sequences of the 3 CDRs of the heavy chain variable region of bevacizumab are as follows:
HCDR1: GYTFTNYG (SEQ ID NO: 63)
HCDR2: INTYTGEP (SEQ ID NO: 64)
HCDR3: AKYPHYYGSSHWYFDV (SEQ ID NO: 65)

The amino acid sequences of the 3 CDRs of the light chain variable region of bevacizumab are as follows:
LCDR1: QDISNY (SEQ ID NO: 66)
LCDR2: FTS (SEQ ID NO: 67)
LCDR3: QQYSTVPWT (SEQ ID NO: 68)
SEQ ID NO: 69 nucleic acid sequence of CP004(hG1TM) heavy chain 
GAAGTGCAGCTGGTCGAGTCTGGGGGAGGGCTGGTGCAGCCCGGC
GGGTCACTGCGACTGAGCTGCGCAGCTTCCGGATTCGCCTTTAGCTC
CTACGACATGTCCTGGGTGCGACAGGCACCAGGAAAGGGACTGGAT
TGGGTCGCTACTATCTCAGGAGGCGGGAGATACACCTACTATCCTGA
CAGCGTCAAGGGCCGGTTCACAATCTCTAGAGATAACAGTAAGAAC
AATCTGTATCTGCAGATGAACAGCCTGAGGGCTGAGGACACCGCAC
TGTACTATTGTGCCAACCGCTACGGGGAAGCATGGTTTGCCTATTGG
GGGCAGGGAACCCTGGTGACAGTCTCTAGTgccagcaccaaagggcccagcgtgt
ttcctctcgccccctcctccaaaagcaccagcggaggaaccgctgctctcggatgtctggtgaaggactacttcc
ctgaacccgtcaccgtgagctggaatagcggcgctctgacaagcggagtccatacattccctgctgtgctgcaa
agcagcggactctattccctgtccagcgtcgtcacagtgcccagcagcagcctgggcacccagacctacatctg
taacgtcaaccacaagccctccaacaccaaggtggacaagaaagtggagcccaaatcctgcgacaagacaca
cacctgtcccccctgtcctgctcccgaaGCTGCTggagcccctagcgtcttcctctttcctcccaaacccaa
ggacaccctcatgatcagcagaacccctgaagtcacctgtgtcgtcgtggatgtcagccatgaggaccccgag
gtgaaattcaactggtatgtcgatggcgtcgaggtgcacaacgccaaaaccaagcccagggaggaacagtaca
actccacctacagggtggtgtccgtgctgacagtcctccaccaggactggctgaacggcaaggagtacaagtg
caaggtgtccaacaaggctctccctgcccccattgagaagaccatcagcaaggccaaaggccaacccaggga
gccccaggtctatacactgcctccctccagggacgaactcaccaagaaccaggtgtccctgacctgcctggtca
agggcttttatcccagcgacatcgccgtcgagtgggagtccaacggacagcccgagaataactacaagaccac
ccctcctgtcctcgactccgacggctccttcttcctgtacagcAAACTGACCGTGGATAAGTCC
 
AGATGGCAGCAGGGCAATGTCTTTTCATGTTCCGTGATGCACGAGGC
ACTGCACAACCACTATACCCAGAAGTCTCTGAGTCTGTCACCAGGAA
AAGGAGGAGGAGGCTCTGGAGGAGGCGGAAGTGGAGGCGGAGGAT
CAGGAGGGGGAGGATCTCAGGTGCAGCTGGTCGAATCCGGAGCCGA
GGTGAAGAAACCCGGCGCTTCCGTGAAGGTCTCTTGCAAAGCATCA
GGCTACAGCTTCACAGGGTATACTATGAACTGGGTGCGGCAGGCACC
TGGACAGTGTCTGGAATGGATCGGCCTGATTAACCCATACAACAACA
TCACTAACTACGCCCAGAAGTTCCAGGGCCGGGTGACTTTTACCGTG
GACACTAGCATTTCCACCGCTTACATGGAGCTGAGTCGGCTGAGATC
AGACGATACCGGCGTGTATTTTTGCGCAAGGCTGGATTACAGAAGTT
ATTGGGGACAGGGAACACTTGTTACAGTCTCTGCTGGAGGAGGCGG
ATCTGGAGGAGGAGGATCTGGCGGAGGAGGCAGTGGAGGAGGAGG
ATCACAGGCTGTGGTTACTCAGGAACCAAGCCTGACCGTGAGCCCC
GGAGGCACAGTCACTCTGACCTGTGGGAGCTCCACAGGAGCTGTGA
CCACATCTAACTTCCCTAATTGGGTGCAGCAGAAGCCAGGACAGGC
ACCTCGATCCCTGATCGGGGGAACCAACAACAAGGCCAGCTGGACA
CCCGCCAGATTTTCTGGCAGTCTGCTGGGCGGGAAAGCCGCTCTGA
CCATTAGCGGCGCTCAGCCTGAGGACGAAGCAGAGTACTATTGCGCC
CTGTGGTATAGTAATCATTGGGTGTTCGGGTGTGGGACAAAACTGAC
CGTGCTGAGA
SEQ ID NO: 70 amino acid sequence of CP004(hG1TM) heavy chain (full-length) 
EVQLVESGGGLVQPGGSLRLSCAASGFAFSSYDMSWVRQAPGKGLDW
VATISGGGRYTYYPDSVKGRFTISRDNSKNNLYLQMNSLRAEDTALYYC
ANRYGEAWFAYWGQGTLVTVSSASTKGPSVFPLAPSSKSTSGGTAALG
CLVKDYFPEPVTVSWNSGALTSGVHTFPAVLQSSGLYSLSSVVTVPSSSL
GTQTYICNVNHKPSNTKVDKKVEPKSCDKTHTCPPCPAPEAAGAPSVF
LFPPKPKDTLMISRTPEVTCVVVDVSHEDPEVKFNWYVDGVEVHNAK
 
TKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALH
NHYTQKSLSLSPGKGGGGSGGGGSGGGGSGGGGSQVQLVESGAEVKK
PGASVKVSCKASGYSFTGYTMNWVRQAPGQCLEWIGLINPYNNITNYA
QKFQGRVTFTVDTSISTAYMELSRLRSDDTGVYFCARLDYRSYWGQGT
LVTVSAGGGGSGGGGSGGGGSGGGGSQAVVTQEPSLTVSPGGTVTLTC
GSSTGAVTTSNFPNWVQQKPGQAPRSLIGGTNNKASWTPARFSGSLLG
GKAALTISGAQPEDEAEYYCALWYSNHWVFGCGTKLTVLR (Note: the
underlined parts represent CDR sequences)
SEQ ID NO: 71 nucleic acid sequence of CP004(hG1TM) light chain 
GACATTCAGATGACTCAGAGCCCCTCCTCCATGTCCGCCTCTGTGGG
CGACAGGGTCACCTTCACATGCCGCGCTAGTCAGGATATCAACACCT
ACCTGAGCTGGTTTCAGCAGAAGCCAGGGAAAAGCCCCAAGACACT
GATCTACCGGGCTAATAGACTGGTGTCTGGAGTCCCAAGTCGGTTCA
GTGGCTCAGGGAGCGGACAGGACTACACTCTGACCATCAGCTCCCT
GCAGCCTGAGGACATGGCAACCTACTATTGCCTGCAGTATGATGAGT
TCCCACTGACCTTTGGCGCCGGGACAAAACTGGAGCTGAAGCGAAC
TGTGGCCGCTCCCTCCGTCTTCATTTTTCCCCCTTCTGACGAACAGCT
GAAATCAGGCACAGCCAGCGTGGTCTGTCTGCTGAACAATTTCTACC
CTAGAGAGGCAAAAGTGCAGTGGAAGGTCGATAACGCCCTGCAGTC
CGGCAACAGCCAGGAGAGTGTGACTGAACAGGACTCAAAAGATAG
CACCTATTCCCTGTCTAGTACACTGACTCTGTCCAAGGCTGATTACGA
GAAGCACAAAGTGTATGCATGCGAAGTGACACATCAGGGACTGTCA
AGCCCCGTGACTAAGTCTTTTAACCGGGGCGAATGT
SEQ ID NO: 72 amino acid sequence of CP004(hG1TM) light chain (full-length)

DIQMTQSPSSMSASVGDRVTFTCRASQDINTYLSWFQQKPGKSPKTLIY
RANRLVSGVPSRFSGSGSGQDYTLTISSLQPEDMATYYCLQYDEFPLTF
GAGTKLELKRTVAAPSVFIFPPSDEQLKSGTASVVCLLNNFYPREAKVQ
WKVDNALQSGNSQESVTEQDSKDSTYSLSSTLTLSKADYEKHKVYACE
VTHQGLSSPVTKSFNRGEC

## Claims

1. An anti-CTLA4-anti-PD-1 bispecific antibody, wherein the anti-CTLA4-anti-PD-1 bispecific antibody is preferably used for treating a tumor; preferably, the anti-CTLA4-anti-PD-1 bispecific antibody is administered in combination with component A, wherein the component A is selected from an anti-VEGFR2 monoclonal antibody or an antigen-binding fragment thereof, an anti-VEGF monoclonal antibody or an antigen-binding fragment thereof, or a combination thereof,
wherein
(1) the anti-CTLA4-anti-PD-1 bispecific antibody comprises:
a first protein functional region targeting PD-1 and
a second protein functional region targeting CTLA4,
wherein the first protein functional region is an immunoglobulin, and the second protein functional region is a single chain antibody; or, the first protein functional region is a single chain antibody, and the second protein functional region is an immunoglobulin;
wherein,
the immunoglobulin comprises HCDR1-HCDR3 (preferably HCDR1-HCDR3 set forth in SEQ ID NOs: 27-29, respectively) in a heavy chain variable region set forth in SEQ ID NO: 14 and LCDR1-LCDR3 (preferably LCDR1-LCDR3 set forth in SEQ ID NOs: 30-32, respectively) in a light chain variable region set forth in SEQ ID NO: 16; the single chain antibody comprises HCDR1-HCDR3 (preferably HCDR1-HCDR3 set forth in SEQ ID NOs: 33-35, respectively) in a heavy chain variable region set forth in SEQ ID NO: 2 and LCDR1-LCDR3 (preferably LCDR1-LCDR3 set forth in SEQ ID NOs: 36-38, respectively) in a light chain variable region set forth in SEQ ID NO: 4; or,
the immunoglobulin comprises HCDR1-HCDR3 (preferably HCDR1-HCDR3 set forth in SEQ ID NOs: 33-35, respectively) in a heavy chain variable region set forth in SEQ ID NO: 2 and LCDR1-LCDR3 (preferably LCDR1-LCDR3 set forth in SEQ ID NOs: 36-38, respectively) in a light chain variable region set forth in SEQ ID NO: 4; the single chain antibody comprises HCDR1-HCDR3 (preferably HCDR1-HCDR3 set forth in SEQ ID NOs: 27-29, respectively) in a heavy chain variable region set forth in SEQ ID NO: 14 and LCDR1-LCDR3 (preferably LCDR1-LCDR3 set forth in SEQ ID NOs: 30-32, respectively) in a light chain variable region set forth in SEQ ID NO: 16;
(2) the anti-VEGFR2 monoclonal antibody comprises HCDR1-HCDR3 (preferably HCDR1-HCDR3 set forth in SEQ ID NOs: 53-55, respectively) in a heavy chain variable region set forth in SEQ ID NO: 50 and LCDR1-LCDR3 (preferably LCDR1-LCDR3 set forth in SEQ ID NOs: 56-58, respectively) in a light chain variable region set forth in SEQ ID NO: 52; or
(3) the anti-VEGF monoclonal antibody comprises HCDR1-HCDR3 (preferably HCDR1-HCDR3 set forth in SEQ ID NOs: 63-65, respectively) in a heavy chain variable region set forth in SEQ ID NO: 60 and LCDR1-LCDR3 (preferably LCDR1-LCDR3 set forth in SEQ ID NOs: 66-68, respectively) in a light chain variable region set forth in SEQ ID NO: 62.

2. The anti-CTLA4-anti-PD-1 bispecific antibody according to claim 1, wherein,
(1) an amino acid sequence of the heavy chain variable region of the immunoglobulin is selected from a sequence set forth in SEQ ID NO: 14 or SEQ ID NO: 18, or a variant thereof; an amino acid sequence of the light chain variable region of the immunoglobulin is selected from a sequence set forth in SEQ ID NO: 16 or SEQ ID NO: 20, or a variant thereof; an amino acid sequence of the heavy chain variable region of the single chain antibody is selected from a sequence set forth in SEQ ID NO: 2, SEQ ID NO: 6, SEQ ID NO: 10, SEQ ID NO: 41, or SEQ ID NO: 43, or a variant thereof; an amino acid sequence of the light chain variable region of the single chain antibody is selected from a sequence set forth in SEQ ID NO: 4, SEQ ID NO: 8, SEQ ID NO: 12, SEQ ID NO: 42, or SEQ ID NO: 44, or a variant thereof;
or,
an amino acid sequence of the heavy chain variable region of the immunoglobulin is selected from a sequence set forth in SEQ ID NO: 2, SEQ ID NO: 6, SEQ ID NO: 10, SEQ ID NO: 41, or SEQ ID NO: 43, or a variant thereof; an amino acid sequence of the light chain variable region of the immunoglobulin is selected from a sequence set forth in SEQ ID NO: 4, SEQ ID NO: 8, SEQ ID NO: 12, SEQ ID NO: 42, or SEQ ID NO: 44, or a variant thereof; an amino acid sequence of the heavy chain variable region of the single chain antibody is selected from a sequence set forth in SEQ ID NO: 14 or SEQ ID NO: 18, or a variant thereof; an amino acid sequence of the light chain variable region of the single chain antibody is selected from a sequence set forth in SEQ ID NO: 16 or SEQ ID NO: 20, or a variant thereof;
(2) an amino acid sequence of the heavy chain variable region of the anti-VEGFR2 monoclonal antibody is a sequence set forth in SEQ ID NO: 50 or a variant thereof; an amino acid sequence of the light chain variable region of the anti-VEGFR2 monoclonal antibody is a sequence set forth in SEQ ID NO: 52 or a variant thereof; or
(3) an amino acid sequence of the heavy chain variable region of the anti-VEGF monoclonal antibody is a sequence set forth in SEQ ID NO: 60 or a variant thereof; an amino acid sequence of the light chain variable region of the anti-VEGF monoclonal antibody is a sequence set forth in SEQ ID NO: 62 or a variant thereof,
wherein a sequence of the variant has at least 60%, 70%, 80%, 81%, 82%, 83%, 84%, 85%, 86%, 87%, 88%, 89%, 90%, 91%, 92%, 93%, 94%, 95%, 96%, 97%, 98%, or at least 99% homology to the corresponding sequence.

3. The anti-CTLA4-anti-PD-1 bispecific antibody according to any one of claims 1 to 2, wherein the bispecific antibody is selected from any one of the following (1) - (20):
(1) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 2 or a variant thereof, and an amino acid sequence of the light chain variable region of the single chain antibody is a sequence set forth in SEQ ID NO: 4 or a variant thereof;
(2) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 6 or a variant thereof, and an amino acid sequence of the light chain variable region of the single chain antibody is a sequence set forth in SEQ ID NO: 8 or a variant thereof;
(3) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 10 or a variant thereof, and an amino acid sequence of the light chain variable region of the single chain antibody is a sequence set forth in SEQ ID NO: 12 or a variant thereof;
(4) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 2 or a variant thereof, and an amino acid sequence of the light chain variable region of the single chain antibody is a sequence set forth in SEQ ID NO: 4 or a variant thereof;
(5) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 6 or a variant thereof, and an amino acid sequence of the light chain variable region of the single chain antibody is a sequence set forth in SEQ ID NO: 8 or a variant thereof;
(6) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 10 or a variant thereof, and an amino acid sequence of the light chain variable region of the single chain antibody is a sequence set forth in SEQ ID NO: 12 or a variant thereof;
(7) an amino acid sequence of the heavy chain variable region of the immunoglobulin is a sequence set forth in SEQ ID NO: 2 or a variant thereof, and an amino acid sequence of the light chain variable region of the immunoglobulin is a sequence set forth in SEQ ID NO: 4 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
(8) an amino acid sequence of the heavy chain variable region of the immunoglobulin is a sequence set forth in SEQ ID NO: 2 or a variant thereof, and an amino acid sequence of the light chain variable region of the immunoglobulin is a sequence set forth in SEQ ID NO: 4 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
(9) an amino acid sequence of the heavy chain variable region of the immunoglobulin is a sequence set forth in SEQ ID NO: 6 or a variant thereof, and an amino acid sequence of the light chain variable region of the immunoglobulin is a sequence set forth in SEQ ID NO: 8 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
(10) an amino acid sequence of the heavy chain variable region of the immunoglobulin is a sequence set forth in SEQ ID NO: 6 or a variant thereof, and an amino acid sequence of the light chain variable region of the immunoglobulin is a sequence set forth in SEQ ID NO: 8 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
(11) an amino acid sequence of the heavy chain variable region of the immunoglobulin is a sequence set forth in SEQ ID NO: 10 or a variant thereof, and an amino acid sequence of the light chain variable region of the immunoglobulin is a sequence set forth in SEQ ID NO: 12 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
(12) an amino acid sequence of the heavy chain variable region of the immunoglobulin is a sequence set forth in SEQ ID NO: 10 or a variant thereof, and an amino acid sequence of the light chain variable region of the immunoglobulin is a sequence set forth in SEQ ID NO: 12 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
(13) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 41 or a variant thereof, and an amino acid sequence of the light chain variable region of the single chain antibody is a sequence set forth in SEQ ID NO: 42 or a variant thereof;
(14) an amino acid sequence of the heavy chain variable region of the immunoglobulin is a sequence set forth in SEQ ID NO: 14 or a variant thereof, and an amino acid sequence of the light chain variable region of the immunoglobulin is a sequence set forth in SEQ ID NO: 16 or a variant thereof; an amino acid sequence of the heavy chain variable region of the single chain antibody is a sequence set forth in SEQ ID NO: 43 or a variant thereof, and an amino acid sequence of the light chain variable region of the single chain antibody is a sequence set forth in SEQ ID NO: 44 or a variant thereof;
(15) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 41 or a variant thereof, and an amino acid sequence of the light chain variable region of the single chain antibody is a sequence set forth in SEQ ID NO: 42 or a variant thereof;
(16) an amino acid sequence of the heavy chain variable region of the immunoglobulin is a sequence set forth in SEQ ID NO: 18 or a variant thereof, and an amino acid sequence of the light chain variable region of the immunoglobulin is a sequence set forth in SEQ ID NO: 20 or a variant thereof; an amino acid sequence of the heavy chain variable region of the single chain antibody is a sequence set forth in SEQ ID NO: 43 or a variant thereof, and an amino acid sequence of the light chain variable region of the single chain antibody is a sequence set forth in SEQ ID NO: 44 or a variant thereof;
(17) an amino acid sequence of the heavy chain variable region of the immunoglobulin is a sequence set forth in SEQ ID NO: 41 or a variant thereof, and an amino acid sequence of the light chain variable region of the immunoglobulin is a sequence set forth in SEQ ID NO: 42 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
(18) an amino acid sequence of the heavy chain variable region of the immunoglobulin is a sequence set forth in SEQ ID NO: 43 or a variant thereof, and an amino acid sequence of the light chain variable region of the immunoglobulin is a sequence set forth in SEQ ID NO: 44 or a variant thereof; an amino acid sequence of the heavy chain variable region of the single chain antibody is a sequence set forth in SEQ ID NO: 14 or a variant thereof, and an amino acid sequence of the light chain variable region of the single chain antibody is a sequence set forth in SEQ ID NO: 16 or a variant thereof;
(19) an amino acid sequence of the heavy chain variable region of the immunoglobulin is a sequence set forth in SEQ ID NO: 41 or a variant thereof, and an amino acid sequence of the light chain variable region of the immunoglobulin is a sequence set forth in SEQ ID NO: 42 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof;
and,
(20) an amino acid sequence of the heavy chain variable region of the immunoglobulin is a sequence set forth in SEQ ID NO: 43 or a variant thereof, and an amino acid sequence of the light chain variable region of the immunoglobulin is a sequence set forth in SEQ ID NO: 44 or a variant thereof; an amino acid sequence of the heavy chain variable region of the single chain antibody is a sequence set forth in SEQ ID NO: 18 or a variant thereof, and an amino acid sequence of the light chain variable region of the single chain antibody is a sequence set forth in SEQ ID NO: 20 or a variant thereof,
wherein a sequence of the variant has at least 60%, 70%, 80%, 81%, 82%, 83%, 84%, 85%, 86%, 87%, 88%, 89%, 90%, 91%, 92%, 93%, 94%, 95%, 96%, 97%, 98%, or at least 99% homology to the corresponding sequence.

4. The anti-CTLA4-anti-PD-1 bispecific antibody according to any one of claims 1 to 3, wherein,
an amino acid sequence of the heavy chain of the immunoglobulin is a sequence set forth in SEQ ID NO: 40 or a variant thereof, and an amino acid sequence of the light chain of the immunoglobulin is a sequence set forth in SEQ ID NO: 24 or a variant thereof, wherein a sequence of the variant has at least 60%, 70%, 80%, 81%, 82%, 83%, 84%, 85%, 86%, 87%, 88%, 89%, 90%, 91%, 92%, 93%, 94%, 95%, 96%, 97%, 98%, or at least 99% homology to the corresponding sequence.

5. The anti-CTLA4-anti-PD-1 bispecific antibody according to any one of claims 1 to 4, wherein the first protein functional region is linked to the second protein functional region either directly or via a linker fragment; and/or the heavy chain variable region of the single chain antibody is linked to the light chain variable region of the single chain antibody either directly or via a linker fragment.

6. The anti-CTLA4-anti-PD-1 bispecific antibody according to claim 5, wherein the linker fragment is (GGGGS)n, n being a positive integer; preferably, n is 1, 2, 3, 4, 5, or 6.

7. The anti-CTLA4-anti-PD-1 bispecific antibody according to any one of claims 1 to 6, wherein the numbers of the first and second protein functional regions are each independently 1, 2, or more.

8. The anti-CTLA4-anti-PD-1 bispecific antibody according to any one of claims 1 to 7, wherein the single chain antibody (preferably the heavy chain variable region) is linked to the heavy chain of the immunoglobulin, preferably linked to the C terminus of the heavy chain of the immunoglobulin.

9. The anti-CTLA4-anti-PD-1 bispecific antibody according to any one of claims 1 to 8, wherein,
the immunoglobulin is of human IgG1 subtype;
wherein, according to the EU numbering system, the immunoglobulin comprises a heavy chain constant region having an A mutation selected from the following mutations:
L234A and L235A; or
L234A and G237A; or
L235A and G237A; or
L234A, L235A, and G237A; and
according to the EU numbering system, the immunoglobulin comprises a heavy chain constant region having a B mutation selected from one or more of the following mutations:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A; or
or, according to the EU numbering system, the immunoglobulin comprises a heavy chain constant region having a combination of an A mutation and a B mutation;
the heavy chain constant region of the anti-VEGFR2 monoclonal antibody or the anti-VEGF monoclonal antibody is Ig gamma-1 chain C region, ACCESSION P01857; the light chain constant region is Ig kappa chain C region, ACCESSION P01834.

10. The anti-CTLA4-anti-PD-1 bispecific antibody according to any one of claims 1 to 9,
wherein, the bispecific antibody comprises:
a first protein functional region targeting PD-1 and
a second protein functional region targeting CTLA4,
the number of the first protein functional region is 1, and the number of the second protein functional region is 2;
wherein the first protein functional region is an immunoglobulin, and the second protein functional region is a single chain antibody;
the heavy chain of the immunoglobulin has an amino acid sequence set forth in SEQ ID NO: 40 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto, and the light chain has an amino acid sequence set forth in SEQ ID NO: 24 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto;
the heavy chain variable region of the single chain antibody has an amino acid sequence set forth in SEQ ID NO: 43 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto, and the light chain variable region of the single chain antibody has an amino acid sequence set forth in SEQ ID NO: 44 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto;
the single chain antibody is linked to the C terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different;
preferably, the first linker fragment and the second linker fragment each have an amino acid sequence independently selected from SEQ ID NO: 25 and SEQ ID NO: 26;
preferably, amino acid sequences of the first linker fragment and second linker fragment are set forth in SEQ ID NO: 26,
preferably, the light chain of the anti-PD-1/CTLA-4 bispecific antibody has a sequence set forth in SEQ ID NO: 72, and the heavy chain of the anti-PD-1/CTLA-4 bispecific antibody has a sequence set forth in SEQ ID NO: 70;
preferably, the antigen-binding fragment is selected from Fab, Fab', F(ab')₂, Fd, Fv, dAb, Fab/c, complementarity determining region (CDR) fragment, single chain antibody (e.g., scFv), bivalent antibody, or domain antibody.

11. A pharmaceutical composition for treating a tumor (particularly a malignant tumor), comprising an effective amount of the anti-CTLA4-anti-PD-1 bispecific antibody according to any one of claims 1-10 and the component A as defined in any one of claims 1-10, wherein the mass ratio of the anti-CTLA4-anti-PD-1 bispecific antibody and the component A is preferably selected from (1:5)-(5:1), such as 1:5, 1:4, 1:3, 1:2, 1:1, 2:1, 3:1, 4:1, or 5:1; preferably, the pharmaceutical composition is in the form of a solid or liquid; optionally, the pharmaceutical composition further comprises one or more chemotherapeutic drugs (preferably, the chemotherapeutic drug is an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel (e.g., albumin-bound paclitaxel, liposome paclitaxel, and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent).

12. Use of the anti-CTLA4-anti-PD-1 bispecific antibody according to any one of claims 1-10 in preparing a medicament or a kit for preventing and/or treating a tumor (particularly a malignant tumor), wherein the medicament or the kit preferably further comprises the component A as defined in any one of claims 1-10; optionally, the medicament or the kit further comprises one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel, albumin-bound paclitaxel, liposome paclitaxel, docetaxel, etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent).

13. A method for preventing and/or treating a tumor (particularly a malignant tumor), comprising administering to a subject a therapeutically effective amount of the anti-CTLA4-anti-PD-1 bispecific antibody according to any one of claims 1-10 in combination with the component A as defined in any one of claims 1-10, and more preferably further in combination with one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel (e.g., albumin-bound paclitaxel, liposome paclitaxel, and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent), wherein the anti-CTLA4-anti-PD-1 bispecific antibody, the component A, and the anti-tumor chemotherapeutic drug are preferably administered simultaneously or sequentially.

14. A kit for preventing and/or treating a tumor (particularly a malignant tumor), comprising (a) a first pharmaceutical composition comprising the anti-CTLA4-anti-PD-1 bispecific antibody according to any one of claims 1-10 as an active ingredient; (b) a second pharmaceutical composition comprising the component A as defined in any one of claims 1-10 as an active ingredient; and optionally (c) one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an alkylating agent, an antimetabolite, an antibiotic, a plant-based and/or hormonal drug, a platinum-based drug (e.g., cisplatin, carboplatin, and oxaliplatin), adriamycin, cyclophosphamide, paclitaxel (e.g., albumin-bound paclitaxel, liposome paclitaxel, and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent).

15. A unit formulation preferably used for treating a tumor (particularly a malignant tumor), wherein the unit formulation comprises 1-10000 mg (preferably 10-1000 mg, preferably 50-500 mg, 100-400 mg, 150-300 mg, 150-250 mg, or 200 mg) of the anti-CTLA4-anti-PD-1 bispecific antibody according to any one of claims 1-10, 1-10000 mg (preferably 1-1000 mg, preferably 50-500 mg, 100-400 mg, 150-300 mg, 150-250 mg, 200 mg, or 100 mg) of the component A as defined in any one of claims 1-10, and optionally one or more drugs for treating a tumor (preferably, the drug is a chemotherapeutic drug or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a VEGF inhibitor, a PARP inhibitor, a double phosphatidylin-ositol-3-kinase/mTOR inhibitor, and a combination thereof), an antibody-drug conjugate (such as maytansine, monomethyl auristatin E, calicheamicin, esperamicin, and a radioisotope chelator), a T cell expressing a chimeric antigen receptor, an antibody or an antigen-binding fragment thereof, an angiogenesis inhibitor, an antineoplastic, a cancer vaccine, an adjuvant and a combination thereof, an antimetabolite, an antibiotic, a plant-based and/or hormonal drug, a platinum-based drug (e.g., cisplatin, carboplatin, and oxaliplatin), adriamycin, cyclophosphamide, paclitaxel (e.g., albumin-bound paclitaxel, liposome paclitaxel, and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, and/or a fluorouracil antineoplastic agent); wherein the anti-CTLA4-anti-PD-1 bispecific antibody, the component A, and the chemotherapeutic drug are packaged separately.

16. A method for preventing or treating cancer or a tumor, wherein the method comprises administering to a subject in need thereof one or more unit formulations according to claim 15; preferably, the anti-CTLA4-anti-PD-1 bispecific antibody, the component A, and the chemotherapeutic drug in the unit formulation are each administered separately.

17. A single dose unit, preferably used for treating a tumor (particularly a malignant tumor), and comprising 0.1-10000 mg (preferably 1-1000 mg, preferably 50-500 mg, 100-400 mg, 150-300 mg, 150-250 mg, 200 mg, or 100 mg) of the anti-CTLA4 anti-PD-1 bispecific antibody according to any one of claims 1-10 and 0.1-10000 mg (preferably 1-1000 mg, preferably 50-500 mg, 100-400 mg, 150-300 mg, 150-250 mg, 200 mg, or 100 mg) of the component A as defined in any one of claims 1-10.

18. The anti-CTLA4-anti-PD-1 bispecific antibody according to any one of claims 1-10, the pharmaceutical composition according to claim 11, the use according to claim 12, the method according to claim 13, the kit according to claim 14, the unit formulation according to claim 15, or the method according to claim 16, wherein
the tumor is selected from one or more of the following:
cervical cancer (e.g., metastatic cervical cancer), lung cancer such as non-small cell lung cancer (e.g., e.g., squamous non-small cell lung cancer or non-squamous non-small cell lung cancer), esophageal cancer, esophageal squamous cancer, ileocecal adenocarcinoma, ampullate adenocarcinoma, small cell lung cancer, gastric cancer (e.g., advanced gastric cancer, gastric adenocarcinoma, or gastroesophageal junction adenocarcinoma), kidney cancer, endometrial cancer, adrenocortical carcinoma, prostate cancer, thyroid cancer, peritoneal cancer, adenocarcinoma, pancreatic cancer, glioma, head and neck cancer, bone cancer, testicular cancer, leukemia, myeloma, lymphoma, sarcoma, mesothelioma, hepatocellular carcinoma, colon cancer, biliary tract cancer, cholangiocarcinoma, rectal cancer, colon cancer, endometrial cancer, ovarian cancer (e.g., advanced ovarian cancer) or fallopian tube cancer, large cell neuroendocrine cancer, urothelial carcinoma (e.g., upper urothelial carcinoma or bladder cancer), breast cancer, triple-negative breast cancer, peripheral T-cell lymphoma, melanoma, nasopharyngeal cancer, high microsatellite instability (MSI-H) or mismatch repair deficient (dMMR) solid tumors, brain cancer (e.g., aggressive brain cancer, such as glioblastoma), ovarian cancer, squamous cell carcinoma, basal cell carcinoma, adenoma, mucinous or serous cystadenocarcinoma, leiomyosarcoma, rhabdomyosarcoma, chorioepithelioma, malignant hydatidiform mole, malignant sertoli cell-stromal cell tumor, malignant granulocytoma, and dysgerminoma.

19. The anti-CTLA4-anti-PD-1 bispecific antibody according to any one of claims 1-10, the pharmaceutical composition according to claim 11, the use according to claim 12, the method according to claim 13, the kit according to claim 14, the unit formulation according to claim 15, or the method according to claim 16, wherein the anti-CTLA4-anti-PD-1 bispecific antibody, the component A, and/or the chemotherapeutic drug is in a form suitable for intravenous injection or intravenous drip infusion, preferably in a liquid form.

20. The method according to claim 13 or 16, wherein the step of administering to the subject an effective amount of the anti-CTLA4-anti-PD-1 bispecific antibody is before or after a surgical treatment and/or before or after a radiotherapy.

21. The method according to claim 13 or 16, wherein,
a unit dose of the anti-CTLA4 anti-PD-1 bispecific antibody is 0.1-100 mg, preferably 1-10 mg per kg body weight; alternatively, a unit dose of the anti-CTLA4-anti-PD-1 bispecific antibody is 10-1000 mg, preferably 50-500 mg, 100-400 mg, 150-300 mg, 150-250 mg, or 200 mg in each subject;
a unit dose of the component A is 0.1-100 mg, preferably 1-10 mg per kg body weight; alternatively, a unit dose of the component A is 10-1000 mg, preferably 50-500 mg, 100-400 mg, 150-300 mg, 150-250 mg, 200 mg, or 100 mg in each subject;
preferably, the dose is administered from twice daily to about once every other day, or once every 3 days, 4 days, 5 days, 6 days, 10 days, 1 week, 2 weeks, 3 weeks, 4 weeks, 5 weeks, or 6 weeks;
preferably, the route of administration is intravenous drip infusion or intravenous injection.
